# EUROPEAN PATENT SPECIFICATION

(11) **EP 0 514 515 B1**
(45) Date of publication and mention of the grant of the patent: **16.04.1997**
(21) Application number: 91920862.9
(22) Date of filing: 03.12.1991
(51) Int. Cl.: A61K 47/48, C07H 15/252

(54) **ANTHRACYCLINE-CONJUGATES**
ANTHRACYCLINKONJUGATE
CONJUGUES D'ANTHRACYCLINE

(30) Priority: 05.12.1990 GB 9026491
(43) Date of publication of application: 25.11.1992
(73) Proprietor: PHARMACIA & UPJOHN S.p.A., 20152 Milano (IT)
(72) Inventor: ANGELUCCI, Francesco, I-20146 Milan (IT); RUGGIERI, Daniella, I-20156 Milan (IT); STEFANELLI, Stefania, I-20143 Milan (IT); SUARATO, Antonino, I-20158 Milan (IT); BERSANI, Laura, I-20089 Basiglio (IT)
(74) Representative: Woods, Geoffrey Corlett
(86) International application number: EP9102284
(87) International publication number: WO9210212

(56) References cited:
- EP-A- 0 014 853
- EP-A- 0 039 060
- EP-A- 0 318 948
- EP-A- 0 328 147
- EP-A- 0 441 218
- WO-A-90/03188
- GB-A- 2 172 594
- File Serveur STN Karlsruhe, File Chemical Abstracts, vol. 94, no. 7, Columbus, Ohio, US; S. ISODA et al.: "Medicinal chemical studies in antiplasmin drugs. VII. Oxa analogs of 4-aminomethylcyclohexanecarboxylic acid", see abstract no. 47233j
- Journal of Controlled Release, vol. 9, no. 1, June 1989, Elsevier Science Publishers B.V., Amsterdam, NL; B. RIHOVA et al.: "Action of polymeric prodrugs based on N-(2-hydroxypropyl)methacrylamide copolymers. I. Suppression of the antibody response and proliferation of mouse splenocytes in vitro", pages 21-32, see page 21, abstract

## Description

The present invention relates to conjugates of therapeutically useful anthracyclines with carriers such as polyclonal and monoclonal antibodies or proteins or peptides of natural or synthetic origin; methods for their preparation, pharmaceutical composition containing them and use thereof in treating certain mammalian tumors. the invention also relates to new anthracycline derivatives and their preparation.

In the last years, many highly cytotoxic anthracyclines have been synthesized. For example, those bearing a morpholino or morpholino substituted ring linked at C-3' position of the sugar moiety have shown promising antitumour activity on experimental murine tumors [see: Bioactive molecules, 55-101, vol 6, Edited by J. William Lown, Elveiser 1988].

The scope of the present invention is to provide anthracycline conjugates with carriers such as monolonal or polyclonal antibodies or proteins or peptides or other carriers of synthetic origin in order to take advantage of the high potency of the anthracyclines, for example the morpholino derivatives, to improve their therapeutic efficacy and to reduce their toxic effects.

EP-A-0318949 describes immunoconjugates of anti-tumour agents comprising acid-cleavable linkers. WO 90/03188 describes acid-cleavable immunoconjugates in which an anthracycline is linked to a protein carrier.

The conjugates of the present invention, characterized by the presence of an acid sensitive acetalic bond, have general formula 1 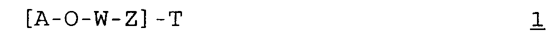 wherein the moiety A-O- is the residue of any anthracycline of formula A-O-H bearing at least one primary or secondary hydroxyl group; a is an integer of from 1 to 30; W is a residue of formula 2 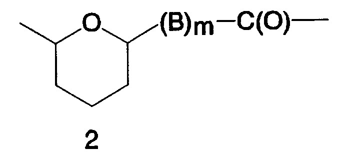 wherein B represents a -CH₂-O-CH₂- group;
Z is a spacer group and T is a carrier moiety.

Preferred Z groups are:
i) -NH-, and T-Z is derived from a carrier of formula T-NH₂ bearing at least one free amino group, or
ii) -NH-[D]_{d}-N=CH- wherein d is an integer of from 0 to 2, [D] represents -NH-CO(CH₂)ₙCO-NH- (n is 2 or 4) and T-Z is derived from a carrier of formula T-CHO bearing at least one formyl group.
iii) the piperazinylcarbonyl moiety or a group of formula

   -NH-[D]_{d}-NH-CO
wherein [D] and a are as defined above
and T-Z is derived from a carrier of formula T-[COOH]ₐ in which a is as above defined.

In the above formula 1 the anthracycline glycoside preferably has the formula 3: 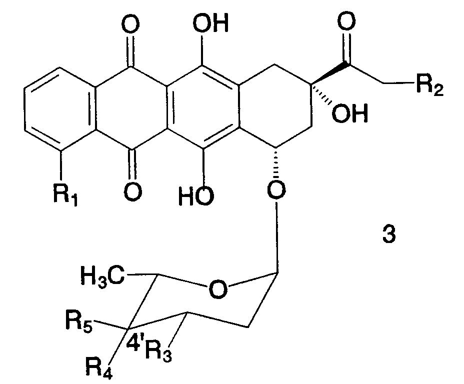 in which:
R₁ is a hydrogen atom, a hydroxy or a methoxy group;
a) R₂ is a hydroxy group and R₄ or R₅ is a hydrogen atom and the other of R₄ and R₅ is a hydroxy group, R₄ is a iodine atom and R₅ is a hydrogen atom or both R₄ and R₅ are hydrogen atoms or
b) R₂ is a hydrogen atom and R₄ or R₅ represents a hydroxy group and the other of R₄ and R₅ is a hydrogen atom; and R₃ is an amino group or represents a nitrogen atom enclosed in a morpholino (MO) or 3-cyano-4-morpholino (CM) or 2-methoxy-4-morpholino (MM) ring in which the nitrogen atom is linked at C-3'

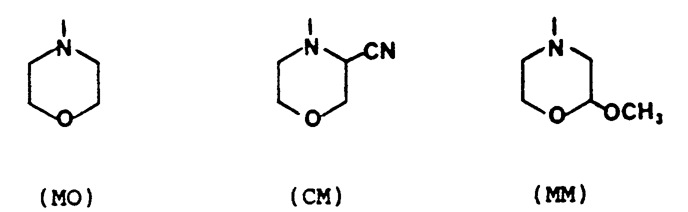

The anthracycline may be linker to the carrier via the hydroxyl group at the C-14 or C-4'-position of the anthracycline compound. In one embodiment, carrier linked to the anthracycline at position C-14, formula 3' represents the moiety A-O-: 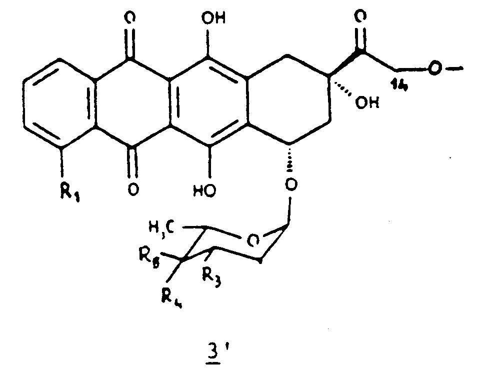 in which R₁ and R₂ are as defined above and R₄ or R₅ is a hydrogen atom and the other of R₄ and R₅ is a hydroxy group or R₄ is a hydrogen or iodine atom and R₅ is a hydrogen atom.

In another embodiment, carrier linked to the anthracycline via the hydroxy group at C-4', formula 3'' represents the moiety A-O-: 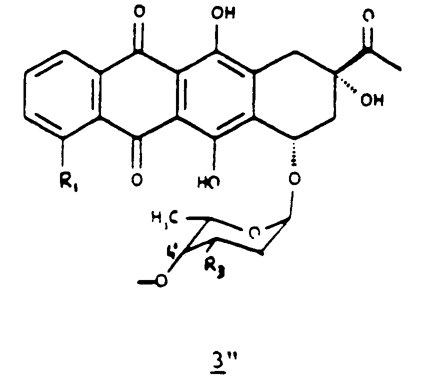 in which R₁ and R₃ are as defined above.

The carrier is typically selected from a polyclonal antibody, or fragment thereof comprising an antigen binding site, capable of binding to a tumor associated antigen; a monoclonal antibody, or fragment thereof comprising an antigen binding site, capable of binding to an antigen preferentially or selectively expressed on tumor cell populations; a peptide or protein capable of preferentially or selectively binding to a tumor cell; and a polymeric carrier.

The carrier portion T-NH₂ or T-[COOH]ₐ of the conjugates is therefore preferably derived from from polyclonal antibodies raised against tumor associated antigens; or from monoclonal antibodies binding to antigens preferentially or selectively espressed on tumor cell populations; or from natural or recombinant peptides or proteins or growth factors preferentially or selectively binding to tumor cells; or from natural or synthetic polymeric carriers such as polylysine, polyglutamic acid, polyaspartic acid and their analogues and derivatives, or such as dextran or other polymeric carbohydrate analogues and their derivatives; or from synthetic copolymers such as those derived from N-(2-hydroxypropyl)methacrylamide (HPMA) [see: J.Kopecek, Macromolecules. H.Benoit & P.Rempp, Ed.: 505-520 (1982) Pergamon Press. Oxford, England); or from poly(aminoacid) copolymers such as poly(GluNa,Ala,Tyr) which are useful as targetable drug-carriers for lung tissue [R.Duncan et al., Journal of Bioactive and Compatible Polymers, Vol 4, July 1989]. The carrier portion may be also derived from portions of the above mentioned carriers, such as the Fab or the F(ab')₂ fragments of the antibodies, or such as portions of the above mentioned peptides or proteins obtained through recombinant DNA techniques.

Representative examples of the above mentioned antibodies and of respective possible therapeutic applications are:
- -anti: T-cell antibody T101 [Royston,I. et al., J.Immunol. 1980, 125, 725]
- -anti: CD5 antibody OKT1 (Ortho) ATCC CRL 8000 (cronic lymphocitic leukemias)
- -anti: trasferrin receptor antibody OKT9 (Ortho) ATCC CRL 8021 (ovaric and other tumors)
- -anti: melanoma antibody MAb 9.2.27 (Bumol,T.F. et al., Proc.Natl.Acad.Sci.USA 1982, 79, 1245) (melanomas)
- -anti: carcinoma markers antibody such as:
-anti-CEA 1116 NS-3d ARCC CRL 8019
-anti alpha-fetoprotein OM 3-1.1 ATCC HB 134 (also hepatomas)
-791T/36 [Embleton, M. J. et al., Br. J. Cancer 1981, 43, 582] (also osteogenic sarcoma)
-B 72.3 [U.S. Pat. No. 4,522,918 (1985)] (colorectal carcinomas and other tumors)
- -anti: ovarian carcinoma antibody OVB 3 ATCC HB 9147
- -anti: breast carcinoma antibody (HMGF antigen) [Aboud-Pirak, E. et al., Cancer Res. 1988, 48, 3188]
- -anti: bladder carcinoma 1G3.10 [Yu,D.S. et al., Eur.J.Urol. 1987, 13, 198]
Representative examples of the above mentioned growth factors and proteins of natural or recombinant origin are FGF, EGF, PDGF, TGF-α, α-MS, Interleukines, Interferones, TNF, melanotropin (MSH), etc.
The carrier T-CHO is preferably derived from polyclonal or monoclonal antibodies having the carbohydrate moiety, preferentially located in the Fc region, selectively oxidized to aldehyde groups by means of chemical or enzymatic methods, as described in U.S.Pat. No.4,671,958 (Jun. 9, 1987). The carrier T-CHO may be also derived from the formylation or from the oxidation of suitable polymeric carriers, or from the oxidation to aldehyde groups of the carbohydrate residues of suitable glycoproteins.

The invention further provides a method for preparing a compound of formula 1 which comprises:
a) converting a derivative of formula 4: 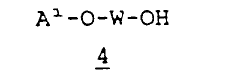 wherein A¹-O- is the residue of any anthracycline bearing at least one primary or secondary hydroxyl group and having the amino group of the sugar moiety protected or replaced by a morpholino derivative and W is as above defined, into an activated derivative of the formula 5: 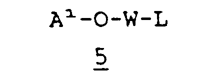 wherein A¹-O- and W have the same meanings as above defined and L represents an activating group for making an amidic linkage; and
b)
   (i) condensing the resultant compounds of formula 5, as above defined, with a compound of formula T-NH₂ as previously defined; or
   (ii) reacting the activated compound of formula 5 with a derivative of formula NH₂-[D]_{d}-NH₂, optionally deprotecting the resultant derivative of formula 6: 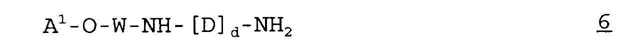 wherein A¹-O-, W, d and [D] are as above defined and condensing it with a compound of formula T-CHO or T-[COOH]ₐ as previously defined or
   (iii) reacting the compound of formula 5 with 1,4-piperazine and condensing the resultant compound of formula 7: 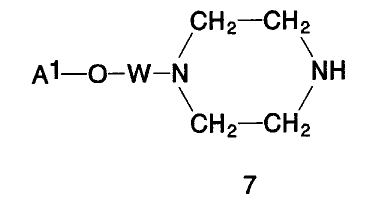 wherein A¹-O- and W are as above defined, with a compound of formula T[COOH]ₐ as defined above, optionally in the presence of a condensing agent to give a conjugate of formula 1, in which the unreacted optionally present activated carboxyl groups may be quenched with a pharmaceutically acceptable amine.

In formula 5 L may be, for example, N-oxysuccinimido, N-oxysulfosuccinimido, 2,4-dinitrophenoxy, 2,3,4,5,6-pentafluorophenoxy or t-butoxy carbonyloxy. The derivative of formula NH₂-[D]_{d}-NH₂ may be, for example, hydrazine (d=0), succinic dihydrazide (d=1 and n=2) or adipic dihydrazide (d=1 and n=4).

For example, an activation method for the conversion of derivatives of formula 4 into N-oxysuccinimidyl derivatives of formula 5 in step (a) of the present process is the reaction of a derivative of formula 4 with N-hydroxysuccinimide or its water soluble 3-substituted sodium sulfonate salt in the presence of N,N'-dicyclohexylcarbodiimide in a solvent such as ethyl acetate or N,N-dimethylformamide. In such a case in formula 5 -L represents the residue: 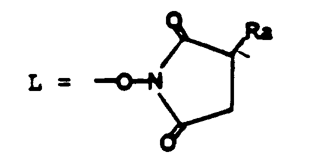 wherein Rₐ represents hydrogen atom or sodium sulphate group.

The condensation methods for preparing conjugates of formula 1, starting from the above mentioned derivatives of formula 5 and a carrier of formula T-NH₂ are carried out in conditions capable of creating covalent linkages of amidic type and compatible with the structure of the carrier. Preferred conditions encompass use of buffered aqueous solutions at pH 7-9.5, temperatures of 4-37°C, for times from some hours to several days.

For example, conditions for the condensation (b) (i) between the compounds of formula 5 and antibodies T-NH₂ are: aqueous 0.1M sodium phosphate and aqueous 0.1M sodium chloride at pH 8 containing a monoclonal antibody at 1 mg/ml, treated with a 30 fold molar excess of a 10% w/v solution of 6 in N,N-dimethylformamide, for 24 hours at 20°C. The conjugate is purified by gel filtration on a Sephadex G-25 column [Pharmacia Fine Chemical, Piscataway, N.J.), eluting with PBS (phosphate-buffered saline).

Methods for preparing conjugates of formula 1, condensing the above mentioned derivatives 6 with a carrier of formula T-CHO, are carried out in conditions capable of creating covalent linkages of hydrazone type and compatible with the structure of the carrier. Preferred conditions encompass use of buffered aqueous solutions at pH 4-7.5, temperature of 4-37°C, for times from some hours to several days.

Conditions for the coupling (b) (ii) between the compounds of formula 6 and antibodies T-CHO are: aqueous 0.1M sodium acetate and aqueous 0.1M sodium chloride at pH 6 containing a monoclonal antibody at 1 mg/ml, treated with a 30 fold molar excess of a 5% w/v solution of 8 in the same buffer, for 24 hours at 20°C. The conjugate is purified by gel filtration as above described.

Method for preparing conjugate of formula 1, by condensing the above mentioned derivatives 6 or 7 and a carrier of formula T[COOH]ₐ are carried out in conditions capable of creating covalent linkages of amidic type and compatible with the structure of the carrier.

Preferred conditions encompass use of buffered aqueous solutions at pH 7-9.5, temperature of 4-37°C, for times from some hours to several days. Other conditions encompass use of dry dimethylformamide or dimethylsulfoxide at room temperature for 1 to 3 hours. Preferred conditions for the condensation between compounds of formula 6 or 7 and an activated carrier of formula T[CO-E]ₐ, in which E represents a suitable activating carbonyl group such as p-nitrophenil, are dry polar solvent such as dimethylformamide containing 5 to 50 mg/ml of compound 6 or 7 treated with an equivalent amount of compound T[CO-E]ₐ for 1 to 24 hours at room temperature.
Herein, the period "from some hours to several days" may be 4 hours to 5 days.

The derivatives of general formula 4, 5, 6, and 7 and their preparation are novel and are within the scope of the present invention. The compounds 4, 5, 6, and 7 are both useful intermediates and/or therapeutically active antitumor agents. The derivatives of formula 4 are prepared by a process which comprises:
(a) condensing an optionally protected anthracycline of the formula A-O-H as defined above, with the proviso that the amino group of the sugar moiety of the anthracycline A-O-H is in the form of a protected amino group, with a dihydropyran carboxylic derivative of formula 8: 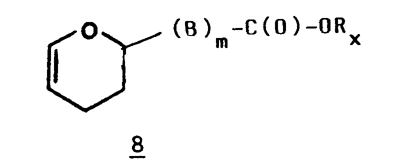 wherein B and m are as defined above and Rx is a protecting group;
(b) removing the or each protecting groups from the resultant intermediates, thereby to form a derivative of formula 4': 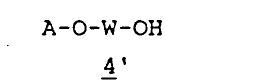 wherein W is as defined above with the proviso that the amino group of the sugar moiety of the anthracycline residue A-O- is in the form of a free amino group; and
(c)
   (i) converting the free amino group of the sugar moiety of the anthracycline of formula 4' into a morpholino derivative or
   (ii) protecting the said free amino group of the said anthracycline of formula 4'.

The intermediate derivative of formula 4' and its preparation are also novel and form part of the present invention. The derivatives are both useful intermediates and/or therapeutically active antitumor agents. The conversion of anthracyclines of general formula A-O-H, suitably protected, into derivatives of general formula 4 as above defined, may be carried out by treatment with a compound of the formula 8 above defined.

The invention further provides a process for the preparation of a derivative of formula 4', which process comprises:
(a) condensing an optionally protected anthracycline of the formula A-O-H as defined above, with the proviso that the amino group of the sugar moiety of the anthracycline A-O-H is in the form of protected amino group, with a dihydropyran carboxylic derivative of formula 8 as defined above; and
(b) removing the or each protecting group from the resultant intermediate.

Preferred conditions for carrying out the reaction of typically protected anthracyclines A-O-H, with compound 8 encompass the use of a sulfonic acid such as p-toluensulfonicacid as catalyst, in anhydrous apolar solvent such as methylene chloride, at room temperature for times from some hours to one day followed by mild alkaline treatment for deblocking the protecting groups.

Derivatives 8 are an enantiomeric mixture. However the etherification reaction with the hydroxyl group of anthracyclines, carried out with acid catalyst, gives only two diastereoisomers, indicated as x and y, having the ring undefined configuration (R or S) both at the acetalic C-2'' position and at another position bearing the -(B)ₘ-C(O)ORx group.

The resulting compound of the formula A-O-W-ORx is converted into compound of the formula 4 as defined above by hydrolysis and deprotection. Some of the starting compound of the formula 8 are new and are within the scope of the present invention.
Preferred derivatives of formula 8 comprise:
8A) 2-ethoxycarbonyl-3,4-dihydro-2H-pyran [m=0, Rx=C₂H₅]
8B) ethyl 2-(3,4-dihydro-2H-pyran-2-yl)methyloxyacetate [B=CH₂-O-CH₂, m=1, Rx=C₂H₅]
8C) methyl 2-(3,4-dihydro-2H-pyran-2-yl)methyloxyacetate [B=CH₂-O-CH₂, m=1, Rx=CH₃]
8D) ethyl 2-(3,4-dihydro-2H-pyran-2-yl)methylthioacetate [B=CH₂-S-CH₂, m=1, Rx=C₂H₅]
8E) ethyl 2-(3,4-dihydro-2H-pyran-2-yl)methylaminoacetate [B=CH₂-NH-CH₂, m=1, Rx=C₂H₅]

Compounds of general formula 8 can be prepared in several ways. For example, they may prepared readily starting from the available 3,4-dihydro-2H-pyran-2-carboxylic acid, sodium salt [CAS 16698-52-5] of formula H or from the corresponding methanol derivative [CAS 3749-36-8] of formula M:

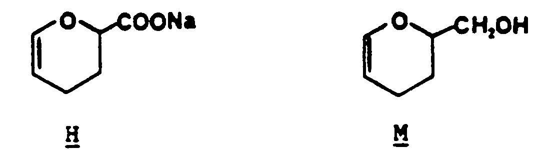

More particularly, compound 8A is prepared by reacting compound H with ethyl iodide in an anhydrous polar solvent, such as dimethylformamide, as described in "Macromolecules" Vol 12, No 1, p 5-9, Jan-Feb 1979. The methyloxyacetate 8B can be prepared by reacting the alcohol M with chloroacetic acid in dry dimethylformamide at 100°C in the presence od two equivalents of sodium or potassium hydroxide, then cooling the mixture and treating it with ethyl iodide.

Compounds of formula 8C-E can be prepared by Wurz condensation of the iodo derivative of M, prepared by displacement of a sulfonic ester of M with sodium iodide, and ethyl 3-iodopropionate, ethyl 4-iodobutanoate or ethyl 7-iodoheptanoate. The methylaminoacetate 8F is prepared by refluxing a sulfonic ester of alcohol M with ethyl glycine in dry toluene for six hours.

Starting anthracyclines of formula 3 for use in the present invention include those bearing a free hydroxyl group, at position C-4' or C-14, such as:
daunorubicin (3a: R₁=OCH₃, R₂=R₅=H,R₃=NH₂, R₄=OH)
4-demethoxydaunorubicin (3b: R₁=R₂=R₅=H, R₃=NH₂, R₄=OH)
4'-epidaunorubicin (3c: R₁=OCH₃, R₂=R₄=H, R₃=NH₂, R₅=OH)
4'-deoxydoxorubicin (3d: R₁=OCH₃, R₂=OH, R₄=R₅=H, R₃=NH₂)
4'-deoxy-4'-jododoxorubicin (3e: R₁=OCH₃, R₂=OH, R₅=H, R₄=J, R₃=NH₂)
and those bearing both secondary and primary hydroxy groups such as:
doxorubicin (3f: R₁=OCH₃, R₂=R₄=OH, R₅=H, R₃=NH₂)
4'-epidoxorubicin (3g: R₁=OCH₃, R₂=R₅=OH, R₄=H, R₃=NH₂), all disclosed in previous patents see: F.Arcamone "DOXORUBICIN" Medicinal Chemistry vol 17, New York, N.Y. 1981.

For example, antracyclines originally bearing a hydroxyl group, for instances those represented by compounds 3a-e, are converted into their N-trifluoroacetyl analogues then reacted with a dihydropyrancarboxylic ester derivatives of general formula 8, as previously described, to afford compounds 4(a-e)(A-Z) in which (a-e) indicate the residue of anthracyclines of formula 3''(a-e) and 3'(d), (A-Z) indicates the residue of general formula 2 derived by condensing a compound of formula 8 to the hydroxyl group at C-4' or at C-14 of the anthracycline.

Anthracyclines bearing both hydroxyl groups at C-4' and C-14 positions, for instances those represented by compounds 3(f,g), may be selectively condensed at C-14 hydroxyl group with compounds of formula 8, after temporary protection of the other hydroxyl group at C-4', to give derivatives of general formula 4 and 4', indicated as 4(f,g)(A-Z) and 4'(f,g)(A-Z), in which (f,g) indicates the residue of anthracycline of formula 3'(f,g) (pyranylation at C-14) or 3''(f,g) (pyranylation at C-4') and (A-Z) have the same meaning as above reported.

Derivatives 4 and 4', as above reported, in which residue -O-W is linked at C-14 hydroxy position are prepared by reacting a compound of formula 8, in the same conditions as previously desribed, with anthracycline derivative bearing protected C-4' hydroxyl group of general formula 9 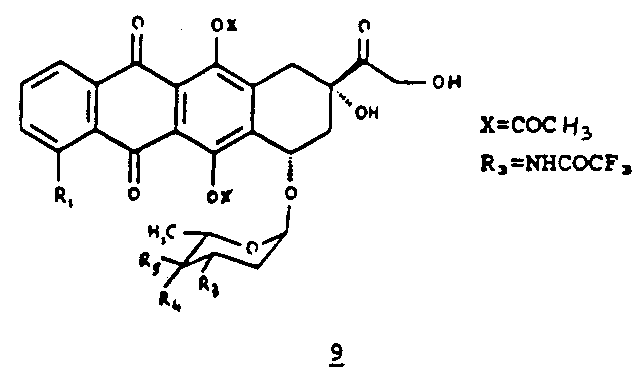 wherein R₁ has the same meaning above reported, R₄ or R₅ is acethoxy group and the other of R₄ and R₅ is hydrogen atom.

For example, N-trifluoroacetyl derivatives of general formula 3 (R₃=NHCOCF₃), such as 3(f,g) are first protected at C-9 and C-14 as 9,14-ethylorthoformate upon reaction with triethyl orthoformate following the procedure described by H.Umezawa et al., J.Antib, vol XXXIII No.12, 1581 (1980), then acylated at phenolic and C-4' hydroxyl groups by using acetic anhydride and pyridine and subsequently deblocked at C-14 hydroxyl position by means of aqueous hydrochloric acid to afford 6,11,4'-tri-O-acetyl-N-trifluoroacetyl doxorubicin derivatives of formula 9 which are condensed with a compound of formula 8 in the same conditions as above reported, to obtain, after deblocking of the phenolic groups by means of morpholine in methanol and deblocking of the hydoxyl group at C-4' with sodium methylate in methanol, compound 10. Finally, treatment of compound 10 with aqueous 0.1N sodium hydroxide affords derivative 4'. The process is illustrated in Scheme I. 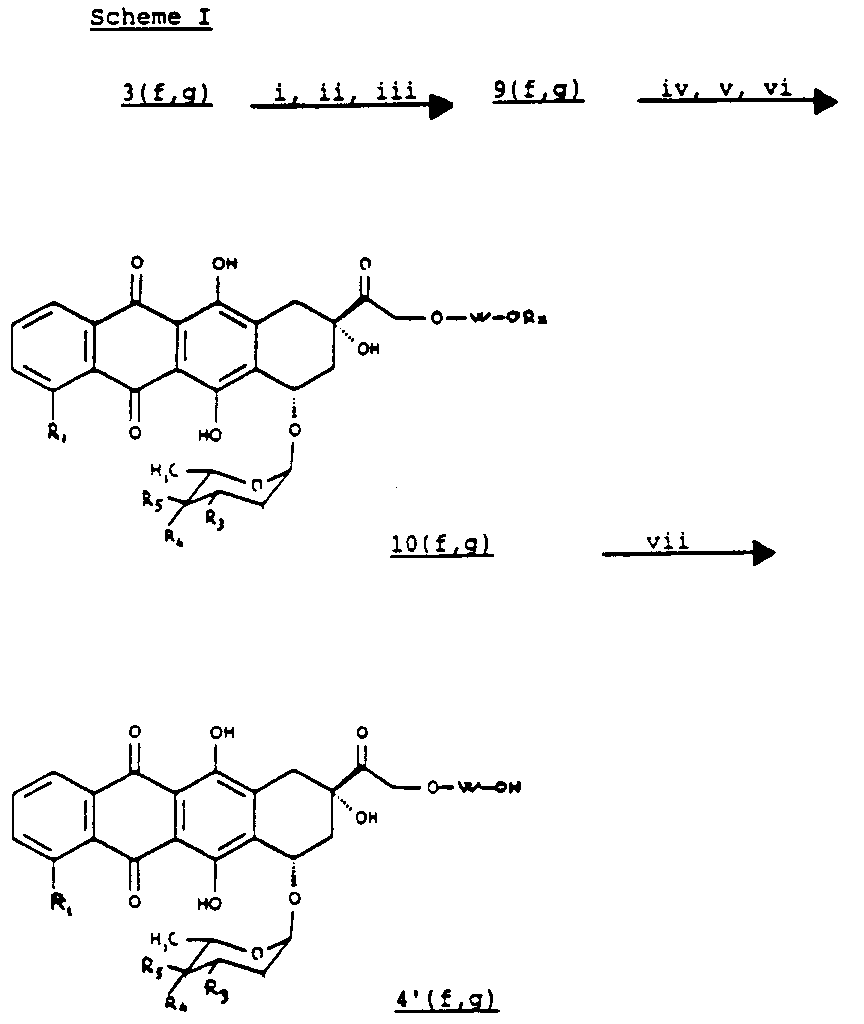 R₃=NHCOCF₃;
R₄=H and R₅=OH or R₄=OH and R₅=H
Reagents & Conditions:
i: CH(C₂H₅O)₃, pTSA, CH₂Cl₂; ii: (CH₃CO)₂O, pyridine;
iii: 0.1N HCl-THF; iv: 8. pTSA. CH₂Cl₂, v: morpholine, CH₃OH: vi: NaOCH₃, CH₃OH; vii: 0.1N NaOH.

Another aspect of the present invention provides the preparation of morpholino (MO) and morpholino derivative (CM) and (MM) of general formula 4(MO), 4(CM) and 4(MM) from the derivatives of general formula 4' (R₃=NH₂), substituted at C-4' or C-14 prepared as above reported, following standard procedures described in previous patents.

More particularly, preparation of 4-morpholino 4(a-g)(A-Z)(MO) and 3-cyano-4-morpholino 4(a-g)(A-Z)(CM) compounds follows the procedure described by E.M.Acton et al., in J.Med.Chem. 1984, 27 638; 2-methoxy-4-morpholino derivatives 4(a-g)(A-Z)(MM) are prepared following the procedure described in US.Pat. N.4,672,057 Jun.9,1987. It should be stressed that the residue -W, in compounds of formula 4'(a-g)(A-Z) does not interfere with the preparation of the above mentioned morpholino derivatives.

According to the process for preparing morpholino derivatives (MO), compounds 4'(a-g)(A-Z), dissolved in water, are first reacted with 2-oxyethyl-dihaldehyde (11), 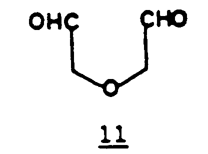 then with sodium cyanoborohydride to afford the morpholino derivative.

By changing the reaction conditions of the cyanoborohydride reduction, for instance by adding sodium cyanide, cyanomorpholino derivative (CN) are recovered.

Preparation of 2-methoxy-4-morpholino derivatives (MM) are performed by treating compounds 4'(a-g)(A-Z), dissolved in water, with 1-methoxy-2,2'-oxydiacetaldehyde (12) 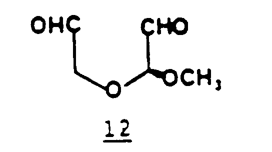 then with sodium cyanoborohydride.

The conjugates of formula 1 of the present invention are useful therapeutic agents since they contain an acetalic bond which releases the parent drug A-O-H upon hydronium-ion-catalyzed hydrolysis or "in vivo" enzymatic cleaveage.

It is well known that in malignant tumors there is a high rate of glycolysis compared to normal tissue. This causes an increase in the production of lactate and thus a decrease of the pH in the tumor [see: H.M.Rauen et al., Z.Naturforsch, Teil B, 23 (1968) 1461]. Also compounds of formula 4, 4', 6 and 7 may release the cytotoxic anthracycline within tumor tissues.

The invention affords a two level specificity of action of the compounds, the first one consisting in a preferential localization of the conjugate in the tumor tissue by means of antigenic recognition, and the second one consisting in a preferential release of the drug in its active form in the tumor tissue by means of preferential acidic cleavage.

The conjugates produced according to the methods described are characterized following different chemico-physical methods.

The retention of the original molecular weight and the lack of aggregate formation is assessed by chromatographic gel filtration procedures (Yu,D.S. et al., J.Urol. 140, 415, 1988) with simultaneous and independent detection of anthracycline and antibody at different wavelenghts, and by gel electrophoretic methods.

The overall charge distribution of the compounds obtained is assessed by chromatographic ion excange methods.

The anthracycline concentration is assessed by spectrophotometric titration against a standard calibration curve obtained from the parent anthracycline.

The protein concentration is assessed by colorimetric assays such as the bicinconic acid assay (Smith,P.K. et al., Anal.Biochem. 150, 76, 1985) or the Bradford dye assay (Bradford,M.M., Anal.Biochem. 72, 248, 1976).

The antigen binding activity retention of the antibodies, after the conjugation procedures, is assessed by an ELISA method (Yu,D.S. et al., J.Urol. 140, 415, 1988) and by cytofluorimetric methods (Gallego,J. et al., Int.J.Cancer 33, 737, 1984).

The evalutation of the retention of cytotoxicity of conjugates in comparison with the parent drug is assessed by a test of inhibition of uptake of ³H-Thymidine by the target cells, after an incubation time long enough to explicate the maximum cytotoxic effect (Dillmann,R.O. et al., Cancer Res. 48, 6097, 1988).

The evalutation of selective cytotoxicity of the conjugates toward an antigen positive in comparison with an antigen negative cell line is assessed by a test of inhibition of uptake of ³H-Thymidine by antigen positive vs. antigen negative cell lines, after a short incubation time (Dillmann,R.O. et al., Cancer Res. 48, 6096, 1988).

The acid sensitivity of the conjugate is evaluated by the above mentioned chromatographic methods after incubation of the compounds in suitable buffered solutions.

Alternatively, radiolabelling of the conjugates in the antibody moiety (¹²⁵I) and/or in the anthracycline moiety (¹⁴C) and HPLC analytical methods are employed for the evalutation of stability in plasma.

The therapeutic effect of the compounds and the improvement of their therapeutic efficacy in comparison with the parent drug, are assessed in animal models of human trasplanted tumors. Nude mice bearing xenografts of human tumors are treated with suitable doses of conjugates, of free drug, of antibody, and of a physical mixture of drug and antibody, at equivalent doses, and the tumor growth is recorded and compared in the different treatment groups.

The immunoglobulin conjugates are formulated as pharmaceutical compositions with a pharmaceutically acceptable carrier or diluent. Any appropriate carrier or diluent may be used. Suitable carriers and diluents include physiological saline solution and Ringers dextrose solutions.

The conjugates of the invention are useful as antitumor agents. A mammal, e.g. a human or animal, may therefore be treated by a method comprising administering thereto a pharmaceutically effective amount of a conjugate of formula 1 as hereinbefore defined. The condition of the human or animal may be ameliorated or improved in this way.

### Detailed Description of the Invention

TLC chromatography are performed with kieselgel plates Merck F₂₅₄ using the following eluting systems (v/v):
- system A:: methylene chloride:methanol (98:2)
- system B:: methylene chloride:methanol:acetic acid:water (80:20:7:3)
- system C:: methylene chloride:methanol (95:5)
- system D:: methylene chloride:acetone (4:1)
- system E:: methylene chloride:methanol:acetic acid (80:20:1)
- system F:: methylene chloride:acetone (9:1)
- system G:: methylene chloride:acetone (95:5)
- system H:: methylene chloride:methanol (90:10)

The invention is further illustrated by the accompanying drawings, in which:

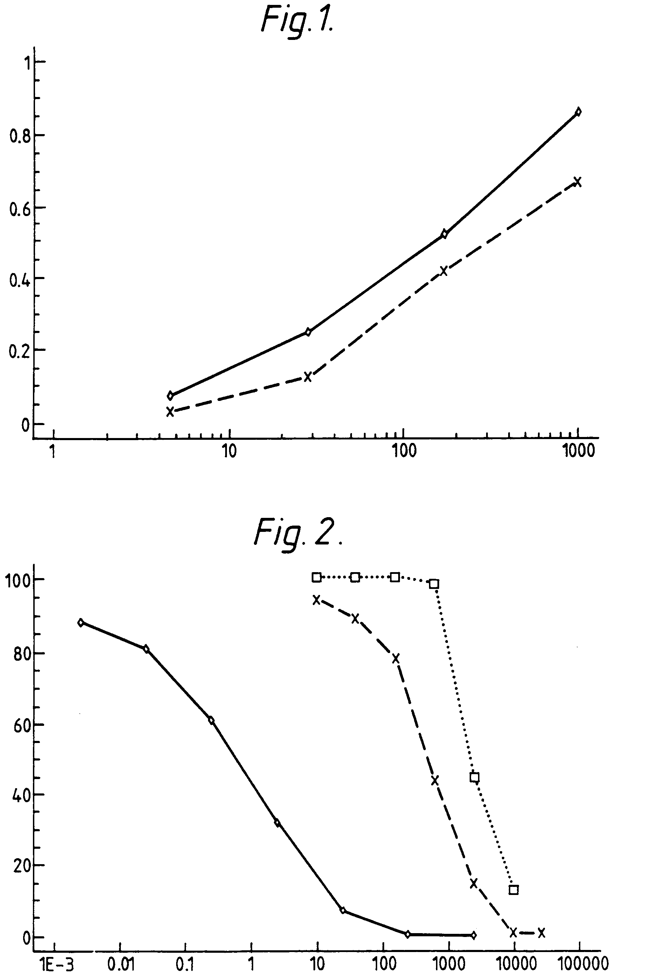

Figure 1 is a graph showing the results of an ELISA, reported in Example 30 which follows, to evaluate the binding to a human melanoma cell line of an anti-transferrin receptor immunoconjugate of the invention 1³ (line X---X---X), and of the parent anti-human transferrin receptor antibody OKT9 (line ◇---◇---◇). In the graph optical absorbance at 495 nm (y axis) is plotted against antibody concentration in ng/ml.
Figure 2 is a graph showing the results of an evaluation, reported in Example 31 which follows, of the selective cytotoxicity of an anti-transferrin receptor immunoconjugate 1³ of the invention (line X---X---X), of an irrelevant control conjugate (line □---□---□) and of the parent free anthracycline drug (line ◇---◇---◇). In the graph (3H) Thymidine incorporation, as a percentage of control (y axis), is plotted against anthracycline concentration in nM (x axis).

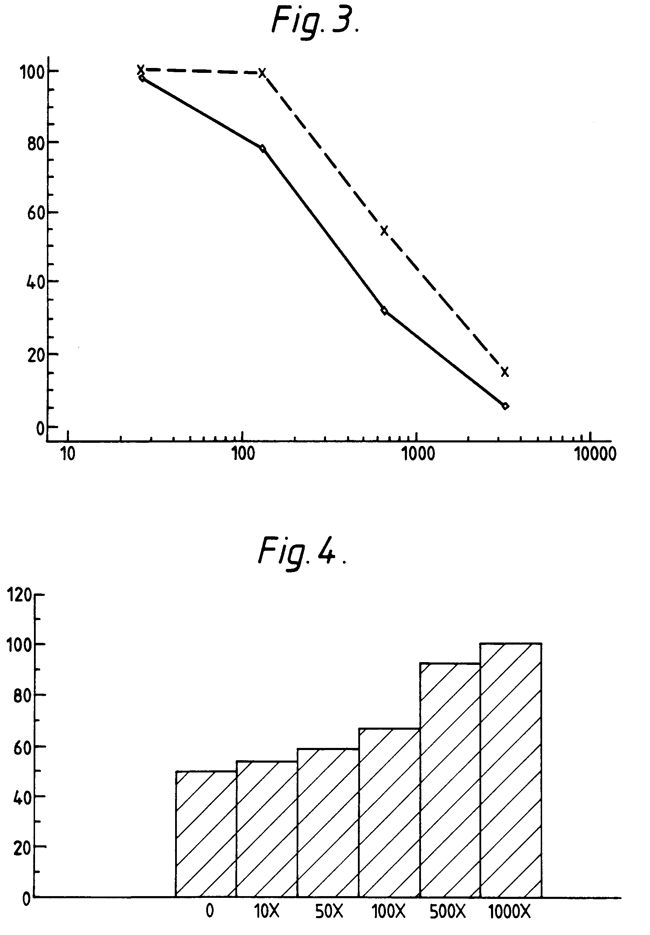

Figure 3 is a graph showing the results of an evaluation, reported in Example 32 which follows, of the inhibition of cytotoxicity of the antitransferrin receptor conjugate 1³ by unconjugated antibody. Line X---X---X = immunoconjugate 1³ + OKT9 antibody and line ◇---◇---◇ = immunoconjugate 1³. The (3H) Thymidine incorporation, as a percentage of control (y axis) is plotted against anthracycline concentration in nM.
Figure 4 depicts the dose-response inhibition of cytotoxicity of the anti-transferrin receptor immunoconjugate 1³ by different excess amounts of unconjugated antibody OKT9, as described in Example 33 which follows. In the graph, (3H) Thymidine incorporation as a percentage of control (y axis) is plotted against free antibody excess (x axis).

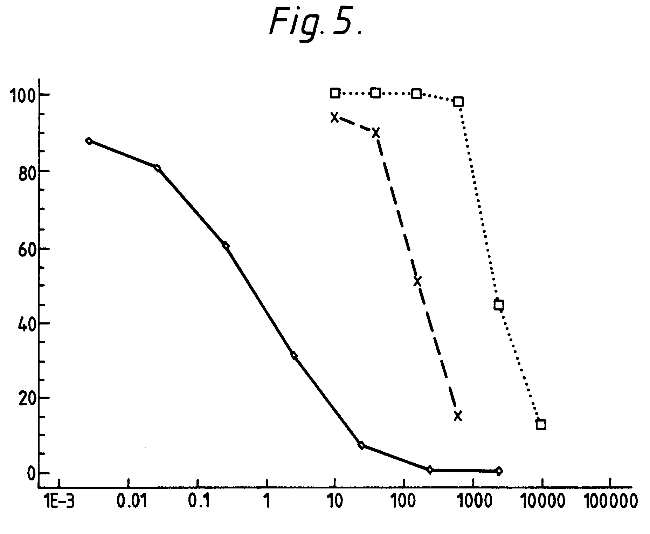

Figure 5 shows the results of an evaluation, reported in Example 34 which follows, of the selective cytotoxicity of the FGF conjugate 1⁷ (line X---X---), of an irrelevant immunoconjugate control 1⁴ (line □---□----□) and of free anthracycline (line ◇---◇---◇). In the graph (3H)Thymidine incorporation, as a percentage of control (y axis), is plotted against anthracycline concentration in nM (x axis).

The following Examples illustrate the invention

### Example 1

### Preparation of Ethyl 2-(3,4-dihydro-2H-pyran-2-yl) methyloxyacetate (8B)

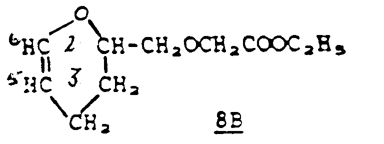

2-methanol-3,4-dihydro-2H-pyran (formula M, 4.2 g, 35 mmol), dissolved in anhydrous methanol (100 ml), was added with sodium hydroxyde (1.6 g, 40 mmol) and gently warmed. Then the solvent was removed in vacuo and the residue was dissolved with dimethylsulfoxide (100 ml). To this solution, warmed at 110°C, was added 2-chloro-sodium acetate (6 g, 1.5 mmol) in dimethylsulfoxide (100 ml) within 2 hours under vigorous stirring. After standing for half an hour, the reaction mixture was cooled, added with triethylamine (5 ml) and ethyl jodide (5 ml) and kept at room temperature overnight. Then the reaction mixture was diluted with water and extracted with ethyl ether. After standard work up, the title compound was purified by chromatography on silicic acid eluting with a mixture of petroleum ether-ether (80:10 v/v). 1.68 g of compound 5 was recoverde as an oil, yield 26%. TLC on Kieselgel plate F₂₅₄ (Merck) using as eluting system the mixture petroleum ether:ether 1:1 by volume) R_{f}=0.7.
¹HNMR (200 MHz, CDCl₃) δ:
1.26 (t, J=7.1 Hz, 3H, CH₂CH₃); 1.6-2.2 (m, 4H, CH₂-3, CH₂-4); 3.64 (d, J=5.2 Hz, 2H, CH₂O); 4.00 (m, 1H, H-2); 4.13 (m, 2H, OCH₂C=O); 4.19 (q, J=7.1 Hz, 2H, CH₂CH₃); 4.65 (m, 1H, H-5); 6.35 (ddd, J=6.2, 2.0, 2.0 Hz, 1H, H-6).

### Example 2

### Preparation of 4'-epi-4'-O-(2-carboxytetrahydropyran-6-yl) daunorubicin: x and y isomers (2''R,6''R and 2''S,6''S).

4'c(A): A¹-O=3''c (R₃=NH₂);

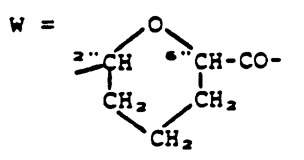

4'-epi-N-trifluoroacetyldaunorubicin (3c: R₃=NHCOCF₃) (3.1g, 5 mmole) was dissolved in anhydrous methylene chloride (500 ml) and treated with 3,4-dihydro-2H-pyran-2-ethoxycarbonyl (8A, 3.4 g, 25 mmole), prepared as described in "Macromulecules" Vol 12, No 1, p 5-9, Jan-Feb 1979, and p-toluensulfonic acid (100 mg) at room temperature under nitrogen. After one hour a chromatographic control showed the formation of two products having, respectively, R_{f} value of 0.53 and 0.36 in system A.
The reaction mixture was washed with aqueous solution of 5% sodium hydrogen carbonate and water, then the organic phase was separated off, dried over anhydrous sodium sulphate, concentrated to small volume under reduced pressure and chromatographed on silicic acid column to afford 4'-epi-4'-O-(2-carboethoxytetrahydropyran-6-yl)-N-trifluoroacetyl daunorubicin derivatives of the title compounds having respectively R_{f} value of 0.53 (1.5 g, yield 40%) and 0.36 (1.28 g, yield 32%) in system A.
Compound R_{f}=0.53; FD-MS: m/e 663 (M+)
¹HNMR (200 MHz, CDCl₃) inter alia δ:
1.31 (COOCH₂CH₃, J=7.1Hz), 1.33 (5'-CH₃, J=6.4Hz), 2.41 (COCH₃), 3.40 (4'-H, J=9.7Hz), 4.07 (4-CH₃O), 4.55 (6''-H), 4.96 (2''-H), 5.30 (7-H), 5.48 (1'-H), 7.92 (NHCOCF₃)
Compound R_{f}=0.36
FD-MS: m/e 663 (M+)
¹HNMR (200 MHz, CDCl₃) inter alia δ:
1.28 (COOCH₂CH₃, J=7.1Hz), 1.38 (5'-CH₃, J=6.4Hz), 2.42 (COCH₃), 3.54 (4'-H, J=8.7Hz), 4.07 (4-CH₃O), 4.42 (6''-H), 5.10 (2''-H), 5.29 (7-H), 5.48 (1'-H), 6.62 (NHCOCF₃).

Compound R_{f}=0.53 (1.4 g, 1.79 mmole) was dissolved with aqueous 0.2N sodium hydroxide under nitrogen and kept at 0°C for eight hours. Then the aqueous solution was adjusted to pH 3 with aqueous 1N hydrogen chloride and extracted repedetly with methylene chloride. The organic phase was washed with water, separated off, dried over anhydrous sodium sulphate and evaporated under reduced pressure to give x, one of the two isomers (x and y)) of the title compound 4'c(A), (0.9 g, yield 79%).
R_{f}=0.60 (system B). FD-MS: m/e 639 (m+)

Compound R_{f}=0.36 (1.1 g, 1.4 mmole) was hydrolized as above described to give, after standard work up, the other isomer y of the title compound 4'c(A), (0.72g, yield 80%). R_{f}=0.45 (system B). FD-MS: m/e 639 (M+).

### Example 3

### Preparation of 4'-epi-4'-O-(2-carboxytetrahydropyran-6yl)-3'-deamino-3'(4-morpholino)daunorubicin: x isomer ( one of the two 2''R,6''R or 2''S,6''S)

4c(A)(MO): A-O=3''c (R₃=MO);

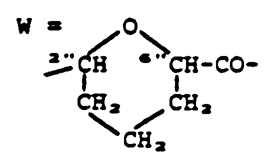

Compound 4'c(A) (x isomer) (1.2 g, 1.8 mmole) prepared as described in Example 2, was dissolved in water (200 ml), adjusted to pH 7.5 with hydrogen sodium carbonate and added with 2-oxyethyl-dihaldehyde (11, 3 g) dissolved in water (20 ml). After one hour a solution of sodium cyanoborohydride (100 mg) in water (6 ml) was added, under stirring at room temperature. After thirty minutes the reaction mixture was brought to pH 6 with an aqueous solution of 5% acetic acid and extracted with n-butylic alchool. The organic phase was washed with water, and the solvent removed under reduced pressure. The residue was purified by chromatography on a column of silic acid using the system methylene chloride: methanol:acetic acid (90:8:2 by volume) to give, after standard work up, the title compound 4c(A)(MO) (0.6 g, yield 45%).
R_{f}=0.70 (system B). FD-MS: m/e 724 (M+).
¹HNMR (200 MHz, CDCl₃) inter alia δ:
1.29 (5'-CH₃, J=6.3Hz), 2.42 (COCH₃), 2.4-2.8 (CH₂-N-CH₂), 2.98 (3'-H), 3.65 (CH₂O-CH₂), 3.40 (4'-H, J=9.0Hz), 4.08 (4-CH₃O), 4.30 (6''-H), 5.00 (2''-H), 5.27 (7-H), 5.51 (1'-H)

### Example 4

### Preparation of N-oxysuccinimidyl derivative of 4'-epi-4'-O-(2-carboxytetrahydropyran-6-yl)-3'-deamino-3'(4-morpholino) daunorubicin: x isomer ( one of the two 2''R,6''R or 2''S,6''S)

5c(A)(MO): A-O=3''c (R₃=MO);

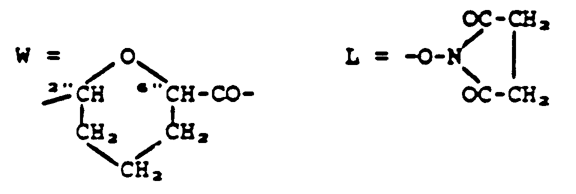

Compound 4c(A)(MO) (0.55 g, 0.7 mmole) prepared as described in Example 3, was dissolved in anhydrous dimethylformamide (25 ml) and treated with N-hydroxysuccinimide (95 mg) and N,N-dicyclohexylcarbodiimide (0.15 g). The mixture was kept at 4°C for two days, then the solvent was removed under reduced pressure. The residue was picked up with little ethyl acetate, filtered off and the solvent removed under reduced pressure. This procedure was performed four times in order to remove the dicyclohexylurea. Finally, the residue was treated with ether from which crystallised 0.45 g, yield 72%, of pure title derivative 5c(A)(MO).
R_{f}=0.45 (system B)
FD-MS m/e 821 (M+)
¹HNMR (200MHz, CDCl₃) inter alia δ:
2.42 (COCH₃), 2.4-2.6 (CH₂-N-CH₂), 2.82 (CO-CH₂-CH₂-CO), 3.5-3.7 (CH₂-O-CH₂), 4.08 (4-CH₃O), 5.13 (2''-H), 5.28 (7-H, 6''-H), 5.52 (1'-H).

### Example 5

### Preparation of 6,11,4'-tri-O-acetyl-N-trifluoroacetyl-doxorubicin (9f)

N-trifluoroacetyl-doxorubicin (3f: R₃=NHCOCF₃) (6.4 g, 10 mmole) was suspended in anhydrous methylene chloride (750 ml) and added with triethylorthoformate (150 ml) and p-toluensulfonic acid (3 g), under stirring at room temperature. After three hours a chromatographic control showed disappearance of the starting material. After standard work up with water, the organic phase was separated off, dryied over anhydrous sodium sulphate and filtered off. The solvent removed in vacuo to afford a crude product which was dissolved in a mixture of pyridine (50 ml) and acetic anhydryde (50 ml) and added with little dimethylaminopyridine (0.5 g). After two hours, the reaction mixture was poured in water and ice (2000 ml). The precipitate was collected on a sintered glass, washed with water, dissolved with tetrahydrofurane (400 ml) and treated with 0.1N aqueous hydrogen chloride (50 ml) overnight at room temperature. Then methylene chloride was added and the organic phase was washed with water, 5% aqueous sodium hydrogen carbonate, and twice with water. After usual work up, the residue was purified by chromatography on a column of silicic acid using the solvent system methylene chloride:acetone (90:10 by volume) to give 5 g, yield 65%, of the title compound 9f.
R_{f}=0.18 (system D)
FD-MS m/e 765 (M+)
¹HNMR (200 MHz, CDCl₃) δ:
1.20 (d, J=6.4Hz, 3H, 5'-CH₃), 1.9-2.0 (m, 2H, 2'-CH₂), 2.1-2.4 (m, 2H, 8-CH₂), 2.19, 2.49, 2.53 (s, 9H, COCH₃ x 3), 2.95 (bt, J=4.2, 1H, CH₂-OH), 3.13 (bm, 2H, 10-CH₂), 3.99 (s, 3H, 4-CH₃O), 4.20 (q, J=6.6Hz, 1H, 5'-H), 4.35 (m, 1H, 3'-H), 4.44 (bs, 1H, 9-OH), 4.74 (m, 2H, CH₂-OH), 5.1-5.3 (m, 3H, 7-H, 1'-H, 4'-H), 6.40 (bd, J=6.4Hz, 1H, NH), 7.3-7.8 (m, 3H, 1-H, 2-H, 3-H).

### Example 6

### Preparation of 14-O-(2-carboxytetrahydropyra-6-yl)doxorubicin, x and y isomers (2''R,6''R and 2''S,6''S).

4'f(A): A¹-O=3'f (R₃=NH₂);

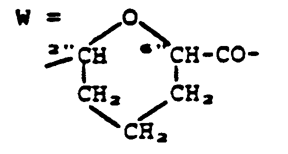

6,11,4'-tri-O-acetyl-N-trifluoroacetyldoxorubicin (10f) (5g, 6.5 mmole), prepared as described in Example 5, was dissolved in anhydrous methylene chloride (600 ml) and treated with 2-ethoxycarbonyl-3,4-dihydro-2H-pyran (8A) (5g, 36 mmole) and p-toluensulfonic acid (500 mg) at room temperature under nitrogen. After two hours the reaction mixture was washed with aqueous solution of 5% sodium hydrogen carbonate and water, then the organic phase was separated off, dried over anhydrous sodium sulphate, concentrated to small volume under reduced pressure and added with petroleum ether. The precipitate was dissolved in methanol (500 ml), added with morpholine (8 ml) and was kept at room temperature for two hours. After that, the reaction mixture was added with acetic acid (10 ml) and extracted with methylene chloride. The organic phase was concentrated to small volume and cromatographed on a silicic acid column using , as eluting system, a mixture of methylene chloride: acetone (95:5 by volume) to obtain the N-trifluoroacetyl 14-O-(2-carbonyltrahydropyran-6-yl)doxorubicin (10f) (4.2 g, yield 82%). R_{f}=0.41 (system D); FD-MS m/e 795 (M+)
¹HNMR (200M, CDCl₃) inter alia δ:
1.3 (5'-CH₃, COOCH₂CH₃), 3.65 (4'-H, J=2.1Hz), 4.08 (4-OCH₃), 4.21 (COOCH₂CH₃, J=7.0Hz), 4.48 (6''-H), 4.88 (9-COCH₂-O), 5.04 (2''-H), 5.27 (7-H), 5.50 (1'-H), 6.69 (NH)

Compound 10f was dissolved at 0°C with 0.1N sodium hydroxyde (500 ml) under nitrogen. After one hour the reaction mixture was brought to pH 6 with acetic acid, extracted with n-butanol and washed with water. The organic phase was separated off and the solvent was removed in vacuo. The residue was picked up with little methylene chloride and added with ether to give the title product 4'f(A) (x,y isomers), 2.9g (yield 66%).
R_{f}=0.5 (system B); FD-MS m/e 671 (M+).

### Example 7

### Preparation of 14-O-(2-carboxytetrahydropran-6-yl)-3'-deamino-3'[2(S)-methoxy-4-morpholino]doxorubicin: x and y isomers (2''R,6''R and 2''S,6''S).

4f(A)(MM): A-O=3'f (R₃=MM);

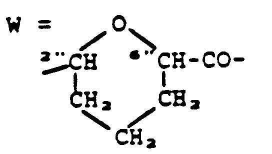

14-O-(2-carboxytetrahydropyran-6-yl)doxorubicin (4'f(A), 1.3 g, 2 mmole), prepared as described in Example 6, was dissolved in water (300 ml) and added with 1-methoxy-2,2'-oxydiacetaldehyde (12) (3.4 g) dissolved in a mixture of acetonitrile (20 ml) and water (20 ml). The mixture was brought to pH 7.5-8.0 with triethylamine and left under stirring, at room temperature, for one hour. Then sodium cyanoborohydride (0.126 g), dissolved in water (10 ml), was added and the reaction mixture was kept at room temperature for 15 minutes. After that, acetone (10 ml) was added and the reaction mixture was added with little acedic acid and extracted with n-butanol. The organic phase was washed with water and the solvent was removed in vacuo. The residue was chromatographed on silicic acid column using as eluting solvent a mixture of methylene chloride:acetic acid:methanol (100:1:7 by volume). The title compound 4f(A)(MM) (0.30 g, yield 26%) was recovered after usual work up.
R_{f}=0.8 (system E), FD-MS m/e 771 (M+)
¹HNMR (200 MHz, CDCl₃) inter alia δ:
1.34 (5'-CH₃), 3.39 (CH₃-O "morpholino"), 4.07 (4-OCH₃), 4.47 (6''-H), 4.58 (CH-OCH₃ "morpholino"), 4.85 (9-COCH₂-O-), 5.04 (2''-H), 5.27 (7-H), 5.59 (1'-H).

### Example 8

### Preparation of N-oxysuccinimidyl derivative of 14-O-(2-carboxytetrahydropyran-6-yl)-3'-deamino-3'[2(S)-methoxy-4-morpholino]doxorubicin: x and y isomers (2''R,6''R and 2''S,6''S).

5f(A)(MM): A-O=3'f (R₃=MM);

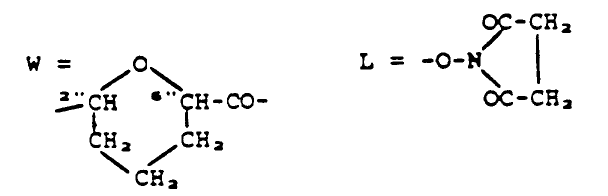

Compound 4f(A)(MM) (0.150 mg), prepared as described in Example 7, was dissolved in anhydrous dimethylformamide (25 ml) and treated with N-hydroxysuccinimide (95 mg) and N,N'-dicyclohexylcarbodiimide (0.15 g) following the procedure described in Example 4. The title compound 5f(A)(MM) was obtained with a yield of 85% (0,15 g).
R_{f}=0.20 (system A); FD-MS m/e 868 (M+).

### Example 9

### Preparation of 14-O-(2-carboxymethyloxymethyl-tetrahydropyran-6-yl)doxorubicin: mixture of isomers 2''R,6''R; 2''S,6''S; 2''S,6''R; 2''R,6''S.

4'f(B): A¹-O=3'f (R₃=NH₂);

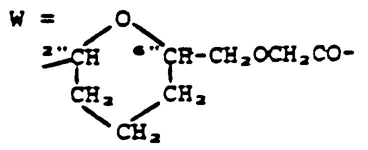

6,11,4'-tri-O-acetyl-N-trifluoroacetyl-doxorubicin (9f, 0.65g, 0.85 mmol), prepared as described in Example 5, was dissolved in anhydrous methylene chloride (50 ml) and treated with compound 8B (0.7 g, 3.5 mmol) and anhydrous p-toluensulfonic acid (0.045 g) under nitrogen at room temperature. After 10 minutes, aqueous satured solution of sodium hydrogen carbonate (20 ml) was added. The organic phase was separated off and washed twice with water, dried over anhydrous sodium sulphate and the solvent was removed under reduced pressure. The oily residue was dissolved in methanol (50 ml) and treated first with morpholine (1.2 ml) for two hours, then with sodium methoxide (0.1 g) for half an hour.
After that, the reaction mixture was brought to pH 7.5 with aqueous 0.1N hydrogen chloride and extracted with methylene chloride. After standard work up, the residue was chromatographed on silicic acid, using methylene chloride as aluting agent, to give 0.4 g (yield 56%) of a protected N-trifluoroacetyl and (2-ethyloxycarbonyl) derivative of the title product. R_{f}=0.31 (system D).
FD-MS: m/e 839 (76, M+); 639 (84); 201 (100)

0.4 g (0.47 mmol) of N-trifluoroacetyl 2-ethyloxycarbonyl derivative above described, was treated with aqueous 0.1N sodium hydroxide (100 ml) at 0°C under nitrogen for 90 minutes. After that, the solution was brought to pH 5 with acetic acid, extracted with water satured of n-butanol and washed with n-butanol satured of water. The solvent was removed under reduced pressure; the residue was piked up with a mixture of methylene chloride-methanol and the title product 4'f(B), 0.32 g (yield 94%), was precipitated by adding ethylic ether and collected on a sintered glass funnel. R_{f}=0.31 (system B).
FD-MS: m/e 716 (23, MH+); 321 (100).

### Example 10

### Preparation of 14-O-(2-carboxymethyloxymethyl-tetrahydropyran-6-yl)-3'-deamino)-3'(4-morpholino)doxorubicin: mixture of isomers 2''R,6''R; 2''S,6''S; 2''S,6''R; 2''R,6''S.

4f(B)(MO): A-O=3'f (R₃=MO);

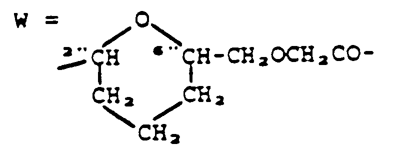

Product 4'f(B) (0.3 g) was dissolved in dry dimethylformamide (40 ml) and treated with triethylamine (0.3 ml) and bis(2-jodoethylether) (11, 2 ml) at 4-5°C.
After 24 hours, the solution was acidified with acetic acid and the solvent was removed under reduced pressure. The residue was treated with aqueos 0.1N sodium hydroxide at 0°C under nitrogen. After 20 minutes, the solution was acidified with acetic acid and extracted with n-butanol, washed with water. The solvent was removed under reduced pressure and the crude material was purified on a silicic acid column eluting with a mixture of methylene chloride/methanol (90:5 by volume) to give the title compound 4f(B)(MO) (0.05 g, yield 23%).
R_{f}=0.48 (system B)
FAB-MS: 757 (M+)

### Example 11

### Preparation of N-oxysuccinimidyl derivative of 14-O-[2-carboxymethyloxymethyl-tetrahydropyran-6-yl]-3'-deamino-3'(4-morpholino)doxorubicin: mixture of isomers 2''R,6''R; 2''S,6''S; 2''S,6''R; 2''R,6''S.

5f(B)(MO): A-O=3'f (R₃=MO);

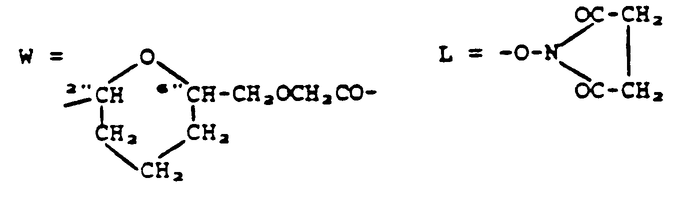

Product 4f(B)(MO) (0.03 g, 0.037 mmole) was dissolved in dry dimethylformamide (3 ml) and added with N-hydroxysuccinimide (0.01 g) and dicyclohexylcarbodiimide (0.02 g). The mixture was kept at room temperature for eight hours, after that, the solvent was removed in vacuo and the residue was chromatographed on silicic acid column using methylene chloride:methanol (95:5) as eluent, to give the title compound 5f(B)(MO) (0.028 g, yield 85%).
R_{f}=0.52 (system H).
FAB-MS: 852 (13, MH+); 200 (100)

### Example 12

### Preparation of 14-O-(2-carboxymethyloxymethyl-tetrahydropyran-6-yl)-3'-deamino-3'[2(S)-methoxy-4-morpholino] doxorubicin: mixture of isomers 2''R,6''R; 2''S,6''S; 2''S,6''R; 2''R,6''S.

4f(B)(MM): A-O=3'f (R₃=MM);

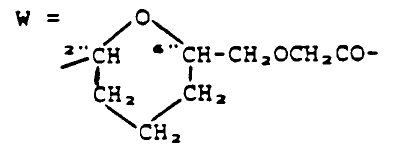

14-O-(2-carboxymethyloxyethyl-tetrahydropyran-6-yl) doxorubicin (4'f(B), 0.3 g), prepared as described in Example 9, was converted into the title compound 4f(B)(MM) by treatment with 1-methoxy-2,2'-oxydiacetaldehyde and reduction with sodiumcyanoborohydride following the same procedure described in Example 7.
R_{f}=0.50 (system B)
FAB-MS: 787

### Example 13

### Preparation of N-oxysuccinimidyl derivative of 14-O-[2-carboxymethyloxymethyl-tetrahydropyran-6-yl]-3'-deamino-3'[2(S)-methoxy-4-morpholino)doxorubicin: mixture of isomers 2''R,6''R; 2''S,6''S; 2''S,6''R; 2''R,6''S.

5f(B)(MM): A-O=3'f (R₃=MM);

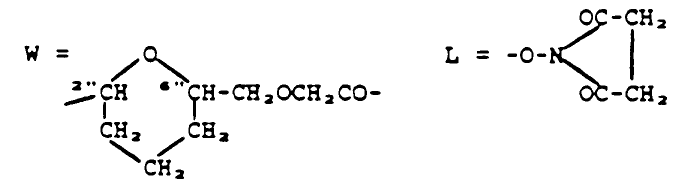

Product 4f(B)(MM), prepared in Example 12, (0.03 g, 0.037 mmole), dissolved in dimethylformamide (3 ml) and added with N-hydroxysuccinimide was transformed into the title compound 5f(B)(MM) (Example 8); R_{f}=0.55 (system H). FAB-MS: 882

### Example 14

### Preparation of 14-O-(6-piperazinecarbonyltetrahydropyran-2-yl)-3'-deamino-3'[2(S)-methoxy-4-morpholiny]doxorubicin: x and y isomers (2''R,6''R ans 2''S,6''S).

7f(A)(MM): A-O=3'f (R₃=MM),

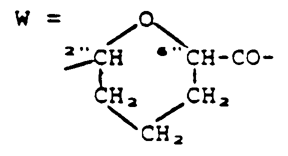

N-oxysuccinimidyl derivative of 14-O-(2-carboxytetrahydropyran-6-yl)-3'-deamino-3'[2(S)-methoxy-4-morpholinyl]doxorubicin 5f(A)(MM), 100 mg], prepared as described in Example 8, was dissolved in anhydrous tetrahydrofurane (20 ml), cooled at 0°C and added with a solution of 1,4-piperazine (50 mg) in anhydrous tetrahydrofurane (2 ml). The reaction mixture was stirred at 0°C for 15 minutes, then was diluted with methylene chloride (100 ml), washed with water (3x50 ml). The organic phase was separated, dryied over anhydrous sodium sulphate and the solvent was removed under reduced pressure. The crude product was chromatographed on silicic acid column using as eluting system a mixture of ethylene chloride/methanol (80/20 by volume). The title compound 7f(A)(MM) (80 mg) was precipitated with ethyl ether.
TLC on Kieselgel Plate (Merck) F₂₅₄, eluting system methylene chloride/methanol (70/30 by volume) R_{f}=0.55
FD-MS: m/z 839

### Example 15

### Preparation of 14-O-(6-hydrazinocarbonyltetrahydropyran-2-yl)-3'-deamino-3'[2(S)-methoxy-4-morpholiny]doxorubicin x and y isomers (2''R,6''R ans 2''S,6''S).

6f(A)(MM): A-O=3'f,

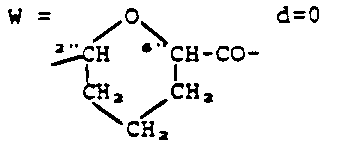

N-oxysuccinimidyl derivative of 14-O-(2-carboxytetrahydropyran-6-yl)-3'-deamino-3'[2(S)-methoxy-4-morpholinyl]doxorubicin 4f(A)(MM), 100 mg], prepared as described in Example 8, was dissolved in anhydrous tetrahydrofurane (20 ml) and added with 1M solution of hydrazine hydrate in isopropanol (5 ml). The mixture was kept at 0°C for 1 hour. After that, methylene chloride was added and the mixture was washed three times mith cold water. The organic layer was separated, dryied over anhydrous sodium sulphate, filtered and the solvent removed under vacuum. The residue was purified on silicic acid column using a mixture of methylene chloride/methanol (99/1 by volume). The title compound 6f(A)(MM) (95 mg) was precipitated with ethyl ether.
TLC on Kieselgel Plate (Merck) F₂₅₄, eluting system methylene chloride/methanol (95/5 by volume) R_{f}=0.23
FD-MS: m/z 785
¹HNMR (200 MHz, CDCl₃): δ:
1.36 (d, J=6.6Hz, 3H, 5'-CH₃); 1.2-2.2 (m, 9H, 3''-CH₂, 4''-CH₂, 5''-CH₂, 2'-CH₂, 8ax-H); 2.3-2.7 (m, 6H, 3'-H, 8e-H, NCH₂CH₂O, NCH₂-CH(OCH₃)O); 2.9-3.4 (m, 2H, 10-CH₂); 3.38 (s, 3H, NCH₂CH(OCH₃)O); 3.55, 3.90 (two m, 2H, NCH₂CH₂O); 3.68 (m, 1H, 4'-H); 3.95 (m, 1H, 5'-H) ; 4.08 (s, 3H, 4-OCH₃); 4.38 (m, 1H, 6''-H); 4.49 (m, 1H, NCH₂CH(OCH₃)O); 4.6-4.9 (m, 2H, 14-C₂); 4.97 (m, 1H, 2''-H); 5.29 (m, 1H, 7-H); 5.54 (m, 1H, 1'-H); 7.39 (d, J=7.6Hz, 1H, 3-H); 7.59 (bd, J=6.8Hz, 1H, CON); 7.78 (t, J=7.6Hz, 1H, 2-H); 8.03 (d, J=7.6Hz, 1H. 1-H); 13.29 (s, 1H, 11-OH); 13.98 (s, 1H, 6-OH).

### Example 16

### Preparation of anti-human melanoma conjugate 1¹^{.}

A-O=3'f (R₃=MM);

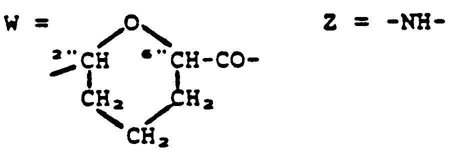

A 10⁻²M solution of compound 5f(A)(MM) (Example 8), in dimethylformamide (38 mcl) was added to 1 ml of a 2 mg/ml solution of purified mouse monoclonal anti-human melanoma antibody Ep1 (P.Giacomini, O.Segatto, P.G.Natali, Int.J.Cancer 39, 1987, 729) in PBS pH 7.5 buffer. The reaction mixture was stirred overnight at room temperature and clarified by centrifugation. The product was isolated by gel filtration chromatography over a Sephadex G-25 column (PD-10, Pharmacia) eluting with PBS pH 7.5. The excluded peak was collected (2 ml) and assayed for anthracycline content spectrophotometrically at 480 nm. The protein content was assayed with a colorimetric protein analysis (BCA, Pierce). The conjugate 1¹ contained 0.98 mg/ml of antibody with an anthracycline:protein ratio of 8.7:1. The chemicophysical profile of the product was determined by HPLC gel filtration analysis with dual wavelenght detection (280 and 480 nm) and by SDS-PAGE. By HPLC, the molecular weight of the product was around 160 kD and the 480 nm anthracycline absorption was in correspondance with the same molecular weight. Proof of the covalent bond formation was obtained by SDS-PAGE, were both the 480 nm anthracycline absorption and the Coomassie-dye protein reaction are located at 160 kD molecular weight.

### Example 17

### Preparation of anti-colon carcinoma conjugate 1².

A-O=3'f (R₃=MM);

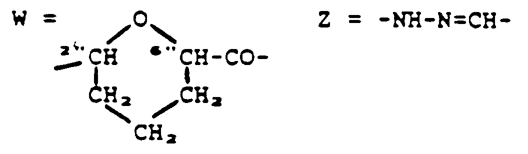

A solution of B72.3 antibody (US Pat.4,522,918, 1985) at 2.6 mg/ml in 0.1M phosphate buffer, pH 6, (1 ml) was treated with 0.1 ml of a 0.1 M solution of NaIO₄ in water, at 4°C in the dark. After 1 hour the product was purified by gel filtration chromatography over a Sephadex G-25 column (PD-10, Pharmacia) eluting with 0.1 M phosphate buffer, pH 6. The protein-containing fraction (1.7 mg, 2 ml) was treated with 30 molar equivalents of a 10⁻²M solution of compound 6f(A)(MM) (Example 15) in dimethylformamide. After 24 hours at 37°C in the dark, the reaction mixture was centrifuged and purified by gel filtration chromatography over a Sephadex G-25 column (PD-10, Pharmacia) eluting with 0.1 M phosphate buffer, pH 7.3. The protein-containing peak was collected (2 ml) and characterized as for Example 16.
The conjugate 1² contained 0.45 mg/ml of antibody with an anthracycline/protein ratio of 1.3/1 and displayed an analytical profile analogous to the conjugate obtained in Example 16.

### Example 18

### Preparation of anti-transferrin receptor conjugate 1³

A-O=3'f (R₃=MM);

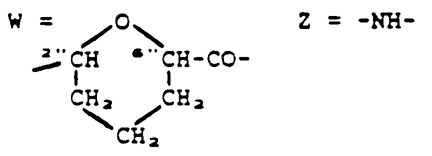

A 10E⁻² M solution of compound 5f(A)(MM) (described in Example 8) in N,N-dimethylformamide (37 mcl) was added to 1 ml of a 2 mg/ml solution of a purified mouse monoclonal anti-human transferrin receptor antibody OKT9 (Sutherland, R., Delia, D., Schneider, C., Newman, R., Kemshead, J.,Greaves, M., Proc. Nat. Acad. Sci. USA, 78 (1981), 4515; ATCC CRL 8021) in 0.1 M NaH2PO4, 0.1 M NaCl, pH 8 buffer. The reaction mixture was stirred overnight at room temperature in the dark and clarified by centrifugation. The title conjugate 1³ was isolated by gel filtration chromatography over a Sephadex G-25 column (PD-10, Pharmacia, Cat. No. 17-0851-01) eluting with PBS pH 7.3 (Gibco, 10X, Cat. N. 042-04200M). The excluded peak was collected and analyzed for anthracycline content (spectrophotometrically at 480 nm) and for protein content (BCA Protein Assay Reagent, Pierce, Cat. 23225). The conjugate contained 1.94 mg/ml of antibody and 80.9 mcg/ml of anthracycline, with an anthracycline : protein molar ratio of 9.5. The analytical profile of the product was evaluated as in Example 16, with similar results.

### Example 19

### Preparation of anti-colon carcinoma conjugate 1⁴

A-O=3'f (R₃=MM);

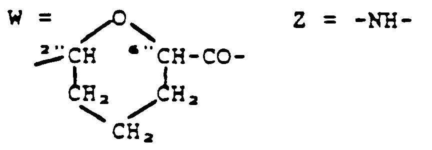

A conjugate of formula 1⁴, containing 0.74 mg/ml of protein and 27.5 mcg/ml of anthacycline, with an anthracycline/protein ratio of 9.1, was obtained with operating as in Example 18 and using in place of OKT9, a solution of B72.3 antibody (2.3 mg/ml) [Schlom, J. et al., US Pat. 4 522 918 (1985)] and 43 mcl of the solution of 5f(A)(MM),

### Example 20

### Preparation of anti-epidermal growth factor conjugate 1⁵

A-O=3'f (R₃=MM);

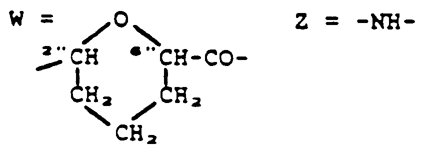

Conjugate 1⁵, containing 0.49 mg/ml of protein and 14.3 mcg/ml of anthracycline, with an anthracycline/protein ratio of 6.9, was obtained with operating as in Example 18 and using in place of OKT9, a solution of an anti-epidermal growth factor receptor antibody (1.15 mg/ml) (BioMacor Cat. No. 6080, Clone 29.2) and 13 mcl of the solution of compound 5f(A)(MM).

### Example 21

### Preparation of anti-transferrin receptor conjugate 1⁶.

A-O=3'f (R₃=MM);

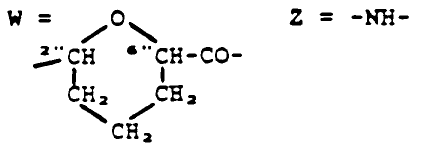

The title compound 1⁶, containing 0.34 mg/ml of protein and 5.3 mcg/ml of anthracycline, with an anthracycline/protein ratio of 3.9, was obtained with operating as in Example 18 and using in place of OKT9 a solution of the anti-transferrin receptor antibody B3/25 (0.5 mg/ml) (Boehringer Cat. No. 1118 048) and 9.4 mcl of the solution of 5f(A)(MM).

### Example 22

### Preparation of FGF conjugate 1⁷.

A-O=3'f (R₃=MM);

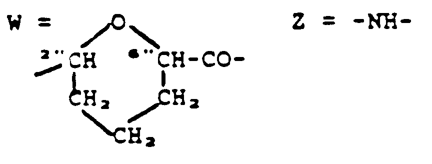

Compound 1⁷ containing 0.77 mg/ml of protein and 117 mcg/ml of anthracycline, with an anthracycline/protein ratio of 3.4, was prepared with operating as in Example 18 and using in place of OKT9, a solution of human recombinant FGF (1.35 mg/ml)(Barr et al, J. Biol. Chem. 263 (1988), 16471) and 48 mcl of the solution of 5f(A)(MM).

### Example 23

### Preparation of anti-transferrin receptor conjugate 1⁸

A-O=3'f (R₃=MO);

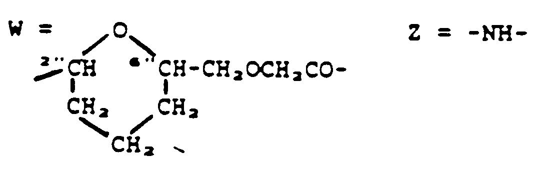

Conjugate 1⁸, containing 0.46 mg/ml of protein and 13 mcg/ml of anthracycline, with an anthracycline/protein ratio of 4.9O was botained with operating as in Example 18 and using 37 mcl of a 10E⁻² M solution of 5f(B)(MO) (Example 11).

### Example 24

### Preparation of anti-colon carcinoma conjugate 1⁹.

A-O=3'f (R₃=MO);

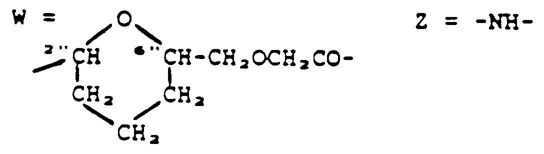

Conjugate of formula 1⁹ containing 0.46 mg/ml of protein and 8.38 mcg/ml of anthracycline, with an anthracycline : protein ratio of 4.5 was obtained with operating as in Example 18 and using in place of OKT9, the anti-colon carcinoma antibody B72.3 and 43 mcl of a 10E⁻² M solution of 5f(B)(MO) (Example 11).

### Example 25

### Preparation of anti-transferrin receptor conjugate 1¹⁰.

A-O=3'f (R₃=MO);

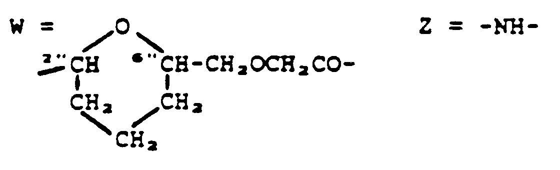

Compound 1¹⁰ containing 0.67 mg/ml of protein and 20.3 mcg/ml of anthacycline, with an anthracycline/protein ratio of 7.6 was obtained with operating as in Example 18 and using 37 mcl of a 10E⁻² M solution of 5f(B)(MM) (Example 13).

### Example 26

### Preparation of anti-colon carcinoma conjugate 1¹¹.

A-O=3'f (R₃=MO);

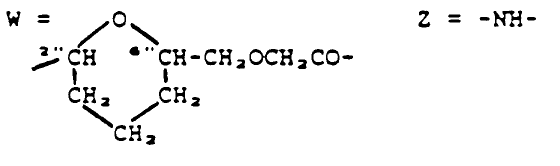

Operating as in Example 18 and using in place of OKT9, the anti-colon carcinoma antibody B72.3 and 43 mcl of a 10E⁻²M solution of compound 5f(B)(MO), was prepared the title compound 1¹¹ containing 0.55 mg/ml of protein and 7.4 mcg/ml of anthracycline, with an anthracycline/protein ratio of 3.2.

### Example 27

### Preparation of conjugate of Poly(Glu.Na,Ala,Tyr) (1:1:1) 1¹²

A-O=3'f (R₃=MM);

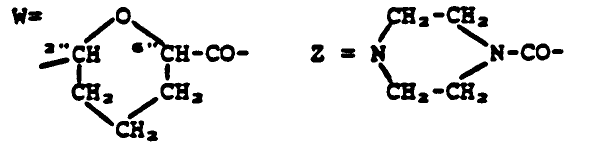

Poly(Glu.Na,Ala,Tyr) (1:1:1) M_{w} 25000-40000 (Sigma), (0.2 g) was dissolved in water (5 ml) under stirring at room temperature. The corresponding free acid was precipitated from the aqueous solution at pH 3 by means of 0.1N HCl. Poly(Glu-OH,Ala,Tyr) (0.17 g), recovered and dryied under vacuum, was dissolved in dry dimethylformamide (10 ml) and added with 14-O-(6-piperazinecarbonyltetrahydropyran-2-yl)-3'-deamino-3'[2(S)-methoxy-4-morpholiny]doxorubicin
[7f(A)(MM), 0.035 g] (described in Example 14) and N-ethoxycarbonyl-2-ethoxy-1,2-dihydroquinoline (EEDQ) (0.08 g). Another aliquot of EEDQ (0.08 g) was added after three hours. The reaction mixture was stirred overnight at room temperature, than was poured in ethyl ether (300 ml). The precipitated was suspended in water (10 ml) and treated with 0.1N NaOH (14 ml); the solution was brought to pH 8.5 with 0.1N HCl and passed on a column of Sephadex G10. The aqueous solution was liophilized to give 0.16 g of the title compound 1¹²
Content of anthracycline (w/w %) calculated as 3'-deamino-3'[2(S)-methoxy-4-morpholinyl]doxorubicin hydrochloride: 10%

### Example 28

### Preparation of conjugate of Poly-L-Glutamic acid 1¹³

A-O=3'f (R₃=MM);

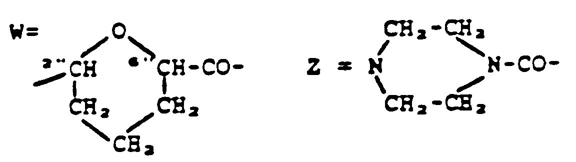

Poly-L-Glutamic acid, M_{w} 2000-15000 (Sigma) (0.1 g) and 14-O-(6-piperazinecarbonyltetrahydropyran-2-yl)-3'-deamino-3'[2(S)-methoxy-4-morpholiny]doxorubicin [7f(A)(MM), 0.03 g], (described in Example 14), were dissolved in anhydrous dimethylformamide (2 ml) and stirred for three hours. After that, N-ethoxy-carbonyl-2-ethoxy-1,2-dihydro-quinoline (EEDQ) (0.03 g) was added. The mixture was kept under stirring overnight, then was poured into a mixture of ethyl ether and petroleum ether. The precipitate was collected on a sintered glass filter, washed with ethyl ether and dissolved with a 2.5% aqueous solution of sodium hydrogen carbonate (8 ml). The solution was passed through a reverse phase column RP-8, 40-63µm (Merck) (30x1.8 cm) and eluted with a mixture of water and acetonitrile. The eluate containing the conjugate was liophilized then collected on a sintered glass filter, washed with methanol and ethyl ether to give the title compound 1¹³ (65 mg). By spectroscopic evaluation, the conjugate contains 16%% (w/w %) of 3'-deamino-3'[2(S)-methoxy-4-morpholinyl]doxorubicin hydrochloride.

### Example 29

### Preparation of conjugate of Poly-L-Glutamic acid 1¹⁴

A-O=3'f (R₃=MM);

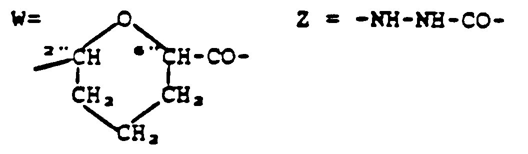

Following the same procedure described in Example 28, Poly-L-Glutamic acid, M_{w} 2000-15000 (Sigma) (0.1 g) and 14-O-(6-hydrazinocarbonyltetrahydropyran-2-yl)-3'-deamino-3'[2(S)-methoxy-4-morpholiny]doxorubicin [6f(A)(MM), 0.05 g], (described in Example 15) were reacted in dimethylformamide in presence of EEDQ. After work up and purification on RP-8, 40-63 µm (Merck) column, 50 mg of the title compound 1¹⁴ was collected.

By spectroscopic evaluation, the conjugate contains 12% (w/w %) of 3'-deamino-3'[2(S)-methoxy-4-morpholinyl]doxorubicin hydrochloride.

### Example 30

### Evaluation of the cell binding activity of the anti-transferrin receptor conjugate 1³ on a human melanoma cell line

- Ref.:: Matsui, M. et al., J. Immunology, 141 (1988) 1410 Bumol, T. F. et al., Antibody-Mediated Delivery Systems, Rodwell, J. D. Ed., (1988) 55 Harper, J. R. et al., Anal. Biochem., 113 (1980) 51

M10 human melanoma cells (ATCC CRL 8021) in RPMI 1640 medium (PBI 12/167B), 10% FCS (Flow Laboratories 29101-54), 1% glutamine were plated in 96 well microtiter plates (Costar 3596) (10000 cells/well). After 16 h at 37°C, cells were washed with PBS, 3% FCS and incubated with different concentrations of the conjugate 1³ (Example 18), or of the OKT9 antibody, in PBS, 3% FCS, 100 mcl, 5-1000 ng/ml for 1 h at 37°C. After three washings with PBS, 3% FCS, 100 mcl of a 1:1500 diluition of horseradish peroxidase conjugated goat anti-mouse IgG (BioRad 172-1011) were added to each well. Plates were then incubated for one hour at 37°C and washed three times with PBS, 3% FCS. Then, 100 mcl of a freshly prepared substrate solution of o-phenylendiamine dihydrochloride (Sigma P6912) (0.5 mg/ml) and H2O2 (0.015%), in H2O, were added to each well. After a 30′ incubation at 37°C the reaction was stopped by the addition of 25 mcl of 4.5 N H2SO4 to each well, and absorbance was read at 495 nm with a BioRad EIA Reader Model 2550. The conjugate displayed a good retention of cell binding activity in comparison with the parent antibody, as shown in Fig. 1.

### Example 31

### Evaluation of the selective cytotoxicity of the anti-transferrin receptor conjugate 1³.

- Ref.:: Dillman, R. O. et al., Cancer Res., 48 (1988) 6097 Ahmad, A. et al., Anticancer Res., 10 (1990) 837

M10 human melanoma cells (ATCC CRL 8021) were plated in 96 well plates (Costar 3596) in 200 mcl RPMI 1640 (PBI 12/167B) containing 10% FCS (Flow Laboratories 29101-54), 1% glutamine, (10000 cells/well) at 37°C. After 18 h, the culture medium was removed and 100 mcl of fresh medium were added. Fifty microliters of different concentrations of the conjugate 1³ and of the relative controls in PBS, 3% FCS, were added to triplicate wells. Plates were incubated for 24h at 37°C, then 0.8 microCurie/well of (3H)thymidine (NEN DuPont NET 355) were added in 20 mcl of medium and incubation was continued at 37°C for 7 h. Cells were harvested with a Dynatech MultiMash 2000 cell harvester onto filter paper (Whatman 1827842), filters were air dried, placed in vials with scintillation fluid (Kontron Analytical Supertron 56920-04010) and counted in a scintillation counter (Kontron Instrument Betamatic).

All the conjugates were substantially less cytotoxic than the parent drug, but the specific anti-transferrin receptor conjugate displayed an effect fivefold superior to an irrelevant control conjugate, is the comparison between the IC50 of the two compounds shows.

Results from a typical experiment are shown in Fig. 2.

### Example 32

### Inhibition of cytotoxicity of the anti-transferrin receptor immunoconjugate 1³ by unconjugated antibody

- Ref.:: Chaundhary, V. K. et al., Nature 339 (1989) 394
Batra, J. K. et al.: Mol. and Cell. Biol. 11 (1991) 2200
Siegal, C. B. et al., J. Biol. Chem. 265 (1990) 16318

M10 human melanoma cells (ATCC CRL 8021) were plated in 96 well plates (Costar 3596) in 200 mcl RPMI 1640 (PBI 12/167B) containing 10% FCS (Flow Laboratories 29101-54), 1% glutamine (10000 cells/well) at 37°C. After 18 h, the culture medium was removed and 50 mcl of fresh medium were added. Then, 50 mcl of a solution of OKT 9 antibody in complete culture medium were added to triplicate wells (final concentration in wells from 7000 to 56 mcg/ml). Control wells were treated with 50 mcl of complete culture medium. Plates were incubated for 1 h at 4°C, then 50 mcl of different concentrations of the conjugate 1³, in complete culture medium, were added (final concentration of antibody in wells from 70 to 0.56 mcg/ml, corresponding to a 1:100 molar excess of free antibody added in the previous step). After 24h at 37°C, 0.8 microCurie/well of (3H)thimidine (NEN DuPont NET 355) in 20 mcl of medium were added, and incubation was continued at 37°C for 7h. Cells were harvested and radioactivity incorporation was evaluated as in Example 31.

As shown in Figure 3, free antibody addition inhibits the cytotoxicity of the immunoconjugate over a range of doses of anthracycline content, thus confirming the receptor mediated effect of the compounds herein described.

Results from a typical experiment are shown in Figure 3.

### Example 33

### Dose-response inhibition of cytotoxicity of the anti-transferrin receptor immunoconjugate 1³ by unconjugated antibody

- Ref.:: Chaundhary, V. K. et al., Nature 339 (1989) 394
Batra, J. K. et al.: Mol. and Cell. Biol. 11 (1991) 2200
Siegal, C. B. et al., J. Biol. Chem. 265 (1990) 16318

M10 cells were plated and incubated for 18h as described in Example 32. After that, the culture medium was removed and 50 mcl of fresh medium were added. Then, 50 mcl of a solution of OKT9 antibody in complete culture medium were added to triplicate wells (final concentration in wells from 2120 to 21.2 mcg/ml). Control wells were treated with 50 mcl of complete culture medium. Plates were incubated for 1h at 4°C. Then, 50 mcl of the conjugate 1³ in complete culture medium, were added (final concentration of antibody in wells 2.12 mcg/ml). Cells were incubated, harvested and radioactivity incorporation evaluated as in Example 31.

As Figure 4 shows, a dose-response related inhibition of conjugate cytotoxicity is effected by the pre-treatement of cells with different excesses of unconjugated antibody, confirming the receptor-mediated cytotoxicity of the compounds.

### Example 34

### Evaluation of the selective cytotoxicity of the FGF conjugate 1⁷.

Operating as in Example 31 and utilizing the conjugate of formula 1⁷, the selective cytotoxicity of the FGF conjugate was demonstrated (Figure 5).

### Example 35

Utilizing the protocol described in Example 31 and different combinations of anthracyclines, linkers, and targeting carriers, different conjugates were prepared, and their cytotoxicity was tested on different cell lines, utilizing non binding conjugates as controls. As Table 1 indicates, specific conjugates are consistently more efficient in inhibiting the growth of tumor cells in comparison with control, non binding, conjugates. The ratio between the IC50 may be taken as an index of the selectivity of the compounds. Thus, from 7% to 38% of a specific conjugate is as cytotoxic as a 100% dose of a control conjugate. The selectivities observed were confirmed from experiments performed with different anthracyclines, linkers, target cells, and for various times of incubation.

**Table 1**

| SELECTIVE CYTOTOXICITY OF ANTHRACYCLINE CONJUGATES | | | | | |
|---|---|---|---|---|---|
| Specific...Control...Target..Incubation...IC50X10E7...IC50.. Conjugate.Conjugate...Cells....Time (h)..Spec./Contr..Ratio. | | | | | |
| 1³ | 1⁴ | M10 | 24 | 5.0/21.0 | 0.23 |
| 1³ | 1⁴ | M10 | 48 | 1.2/ 6.8 | 0.17 |
| 1³ | 1⁴ | M10 | 72 | 1.7/ 5.6 | 0.30 |
| 1³ | 1⁴ | M14 | 48 | 3.9/16.0 | 0.24 |
| 1³ | 1⁴ | M14 | 72 | 5.0/17.0 | 0.29 |
| 1³ | 1⁴ | OVCAR3 | 72 | 4.1/18.0 | 0.23 |
| 1³ | 1⁴ | A431 | 24 | 9.1/40.0 | 0.23 |
| 1⁵ | 1⁴ | A431 | 24 | 18.0/40.0 | 0.45 |
| 1⁷ | 1⁴ | M10 | 24 | 1.5/21.0 | 0.07 |
| 1⁶ | 1⁴ | M10 | 48 | 5.1/10.0 | 0.51 |
| 1⁵ | 1⁴ | M14 | 48 | 11.0/20.0 | 0.55 |
| 1⁸ | 1⁹ | M10 | 24 | 1.0/ 9.0 | 0.11 |
| 1⁸ | 1⁹ | M10 | 72 | 0.4/ 2.3 | 0.17 |
| 1⁸ | 1⁹ | M14 | 24 | 1.2/11.0 | 0.10 |
| 1⁸ | 1⁹ | M14 | 72 | 1.6/12.0 | 0.13 |
| 1¹⁰ | 1¹¹ | M10 | 24 | 2.2/ 9.0 | 0.24 |
| 1¹⁰ | 1¹¹ | M10 | 72 | 3.0/ 8.0 | 0.38 |
| 1¹⁰ | 1¹¹ | M14 | 24 | 5.1/>10.0 | n.d. |
| 1¹⁰ | 1¹¹ | M14 | 72 | 6.0/>10.0 | n.d. |
| Target cells references: M10: human melanoma, ATCC CRL 8021 M14: human melanoma, Natali, P. G. et al., J. Immunol. Meth. 62 (1983), 337 OVCAR-3: human ovaric carcinoma, ATCC HTB 161 A431: human epidermoid carinoma, ATCC CRL 1555 | | | | | |

### Example 36

### Evaluation of compound 1¹⁴.

Compound 1¹⁴ was evaluated "in vivo" against P388 murine Leukemias resistant to Doxorubicin in comparison with 3'-deamino-3'[2(S)-methoxy-4-morpholino]doxorubicin (DMM) and doxorubicin. Data are reported in Table 2.

The conjugate showed similar antitumor activity, but at higher doses, of the free drug.

**Table 2**

| Antitumor activity against P388/DX (Johnson) Leukemia. | | | |
|---|---|---|---|
| compound | P388/DX⁽¹⁾ | | |
| | dose⁽²⁾ (mg/kg) | T/C⁽³⁾ % | TOX |
| 1¹⁴ | 5.2⁽⁴⁾ | 200 | 0/10 |
| Doxorubicin | 16.9 | 100 | 0/10 |
| DMM⁽⁵⁾ | 0.09 | 192 | 0/39 |

| | | | |
|---|---|---|---|
| ⁽¹⁾ 10⁵ cells/mouse (P388/DX, Johnson) transplanted i.v. in CDF1 mice. Treatment on day 1 after inoculation of tumor. | | | |
| ⁽²⁾ Optimal Dose | | | |
| ⁽³⁾ Median survival time % over untreated controls | | | |
| ⁽⁴⁾ Dose of active drug: 3'-deamino-3'[2(S)-methoxy-4-morpholino]doxorubicin (DMM) | | | |
| ⁽⁵⁾ 3'-deamino-3'[2(S)-methoxy-4-morpholino]doxorubicin | | | |

## Claims (Claims for the following Contracting State(s): AT, BE, CH, DE, DK, FR, GB, IT, LI, NL, SE)

1. A conjugate of the formula 1 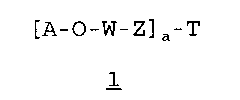 wherein the moiety A-O- is the residue of any anthracycline of formula A-O-H bearing at least one primary or secondary hydroxyl group; a is an integer of from 1 to 30; W is a residue of formula 2 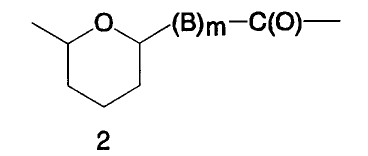 wherein B represents -CH₂-O-CH₂- and m is 0 or 1;
Z is a spacer group and T is a carrier moiety.

2. A conjugate according to claim 1 wherein:
(i) Z represents -NH- and T-Z is derived from a carrier of formula T-NH₂ bearing at least one free amino group, or
(ii) Z is NH-[D]_{d}-N=CH wherein d is an integer of from 0 to 2, [D] represents -NH-CO(CH₂)ₙCO-NH- (n is 2 or 4) and T-Z is derived from a carrier of formula T-CHO bearing at least one formyl group or T[COOH]ₐ wherein a is as defined above or
(iii) Z is a piperazinylcarbonyl moiety or a group of formula -NH-[D]_{d}-NH-CO- wherein [D] and d are as defined above and T-Z is derived from a carrier of formula T-[COOH]ₐ as defined above.

3. A conjugate according to claim 1, wherein the anthracycline bearing at least one primary or secondary hydroxyl group has the formula 3: 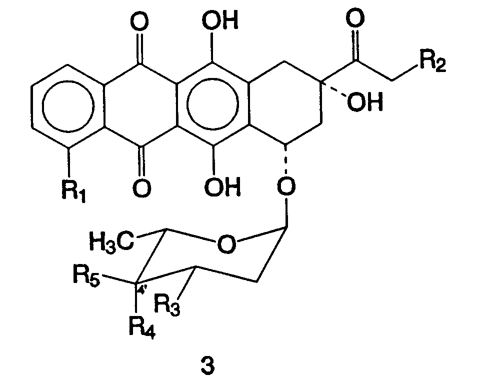 in which:
R₁ is a hydrogen atom, a hydroxy or a methoxy group;
(i) R₂ is a hydroxy group and R₄ or R₅ is a hydrogen atom and the other of R₄ and R₅ is a hydroxy group, R₄ is an iodine atom and R₅ is a hydrogen atom or both R₄ and R₅ are hydrogen atoms or
(ii) R₂ is a hydrogen atom and R₄ or R₅ represents a hydroxy group and the other of R₄ and R₅ is a hydrogen atom;
R₃ is an amino group or represents a nitrogen atom enclosed in a morpholino ring.

4. A conjugate according to claim 3, wherein the moiety A-O- represents: 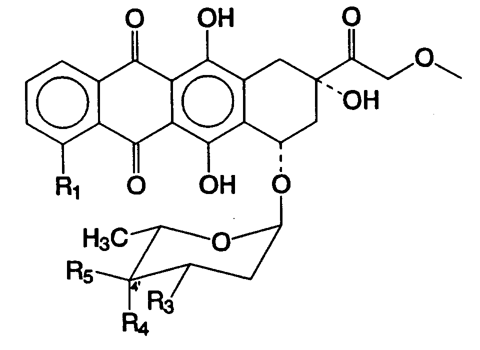 in which R₁ and R₃ are as defined in claim 3 and R₄ or R₅ is a hydrogen atom and the other of R₄ and R₅ is a hydroxy group or R₄ is a hydrogen or iodine atom and R₅ is a hydrogen atom.

5. A conjugate according to claim 3, wherein the moiety A-O- represents: 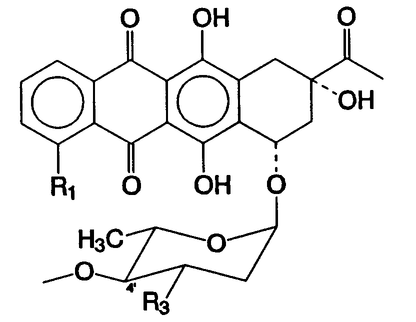 in which R₁ and R₃ are as defined in claim 2.

6. A conjugate according to any one of the preceding claims, wherein the carrier is selected from a polyclonal antibody, or fragment thereof comprising an antigen binding site, capable of binding to a tumor associated antigen; a monoclonal antibody, or fragment thereof comprising an antigen binding site, capable of binding to an antigen preferentially or selectively expressed on tumor cell populations; a peptide or protein capable of preferentially or selectively binding to a tumor cell; and a polymeric carrier.

7. A conjugate according to claim 6, wherein the carrier is selected from an anti-T-cell antibody, an anti-CD5 antibody, an anti-transferrin receptor antibody, an anti-melanoma antibody, an anti-carcinoma marker antibody, an anti-ovarian carcinoma antibody, an anti-breast carcinoma antibody and an anti-bladder carcinoma antibody.

8. A conjugate according to claim 6, wherein the carrier is a growth factor.

9. A process for the preparation of a conjugate of formula 1 as defined in claim 1, which process comprises:
a) converting a derivative of formula 4: 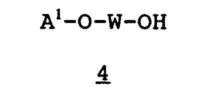 wherein A¹-O- is the residue of an anthracycline bearing at least one primary or secondary hydroxyl group and having the amino group of the sugar moiety protected or replaced by a morpholino group and W is as defined in claim 1, into an activated derivative of the formula 5: 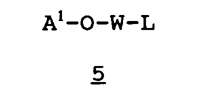 wherein A¹-O- and W are as defined above and L represents an activating group for making an amidic linkage; and
(b)
(i) condensing the resultant compounds of formula 5, as above defined, with a compound of formula T-NH₂ as defined in claim 2 or
(ii) reacting the activated compound of formula 5, with a derivative of formula NH₂-[D]_{d}NH₂, wherein D and d are as defined in claim 2, optionally deprotecting the resultant derivative of formula 6: 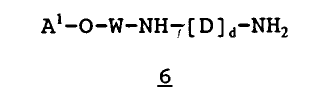 wherein A¹-O-, W, d and [D] are as defined in claim 2 and condensing it with a compound of formula T-CHO or T-[COOH]ₐ as defined in claim 2 or
(iii) reacting the compound of formula 5 with a 1,4-piperazine and condensing the resultant compound of formula 7: 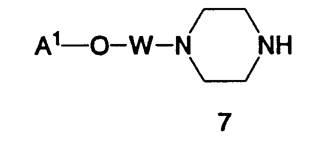 wherein A¹-O- and W are as above defined, with a compound of formula T[COOH]ₐ as defined above, optionally in the presence of a condensing agent to give a conjugate of formula 1, in which the unreacted optionally present activated carboxyl groups may be quenched with a pharmaceutically acceptable amine.

10. A pharmaceutical composition comprising a pharmaceutically acceptable carrier or diluent and, as active ingredient, a conjugate as claimed in any one of claims 1 to 8 or which has been prepared by a process as claimed in claim 9.

11. A derivative of formula 4 as defined in claim 9.

12. A derivative according to claim 11, selected from:
4'-epi-4'-O-(2-carboxytetrahydropyran-6-yl)-3'-deamino-3'-(4-morpholino)-daunorubicin;
14-O-(2-carboxytetrahydropyran-6-yl)-3'-deamino-3'-(2-methoxy-4-morpholino)-doxorubicin; and
14-O-(2-carboxymethyloxymethyl-tetrahydropyran-6-yl)-3'-deamino-3'-(4-morpholino)-doxorubicin.

13. A process for the preparation of a derivative of formula 4 as claimed in claim 11, which process comprises
(a) condensing an optionally protected anthracycline of the formula A-O-H as defined in claim 1, with the proviso that the amino group of the sugar moiety of the anthracycline A-O-H is in the form of a free or protected amino group, with a dihydropyran carboxylic derivative of formula 8: 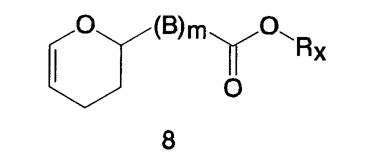 wherein B and m are as defined in claim 1 and Rx is a protecting group;
(b) removing the or each protecting group from the resultant intermediate, thereby to form a derivative of formula 4': 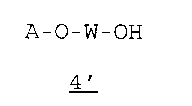 wherein A-O- and W are as defined in claim 1 with the proviso that the amino group of the sugar moiety of the anthracycline residue A-O- is in the form of a free amino group; and
(c)
(i) converting the free amino group of the sugar moiety of the anthracycline of formula 4' into a morpholino derivative, or,
(ii) protecting the said free amino group of the said anthracycline of formula 4'.

14. A derivative of formula 4' as defined in claim 13, with the proviso that when the anthracycline is of formula 3 as defined in claim 3 the anthracycline is not linked to the group -O-W-OH through a hydroxy group at R₄.

15. A derivative according to claim 14, selected from:
4'-epi-4'-O-(2-carboxytetrahydropyran-6-yl)-daunorubicin;
14-O-(2-carboxytetrahydropyran-6-yl)-doxorubicin; and
14-O-(2-carboxymethyloxymethyl-tetrahydropyran-6-yl)-doxorubicin.

16. A process for the preparation of a derivative of formula 4' as claimed in claim 14, which process comprises:
(a) condensing an optionally protected anthracycline of the formula A-O-H as defined in claim 1, with the proviso that the amino group of the sugar moiety of the anthracycline A-O-H is in the form of a free or protected amino group, with a dihydropyran carboxylic derivative of formula 8 as defined in claim 13; and
(b) removing the or each protecting group from the resultant intermediate.

17. A derivative of formula 5 as defined in claim 9.

18. A derivative according to claim 17, selected from:
4'-epi-4'-O-(2-succinimidocarbonyl-tetrahydropyran-6-yl)-3'-deamino-3'-(4-morpholino)-daunorubicin;
14-O-(2-succinimidocarbonyl-tetrahydropyran-6-yl)-3'-deamino-3'-(2-methoxy-4-morpholino)-doxorubicin; and
14-O-(2-succinimidocarbonylmethyloxymethyl-tetrahydropyran-6-yl)-3'-deamino-3'-(4-morpholino)-doxorubicin.

19. A process for the preparation of a derivative of formula 5 as claimed in claim 17, which process comprises converting a derivative of formula 4 as defined in claim 9 into a corresponding said derivative of formula 5.

20. A derivative of formula 6 or 7 as defined in claim 9.

21. A process for the preparation of a derivative of formula 6 or 7 as claimed in claim 20, which process comprises:
(i) reacting a derivative of formula 5 as defined in claim 9 with a derivative of formula NH₂[D]_{d}NH₂ or 1,4-piperazine and, if desired, deprotecting the resultant derivative of formula 6 or 7 thus obtained.

22. A compound of formula: 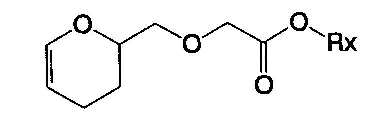 where Rₓ is a protecting group.

23. A derivative according to claim 22, selected from:
2-ethoxycarbonylmethyloxymethyl-3,4-dihydro-2H-pyrane; and
2-methoxycarbonylmethyloxymethyl-3,4-dihydro-2H-pyrane.

24. A conjugate of formula 1 as defined in claim 1 for use as an antitumor agent.

## Claims (Claims for the following Contracting State(s): ES)

1. A process for the preparation of a conjugate of formula 1 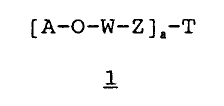 wherein the moiety A-O- is the residue of any anthracycline of formula A-O-H bearing at least one primary or secondary hydroxyl group; a is an integer of from 1 to 30; W is a residue of formula 2 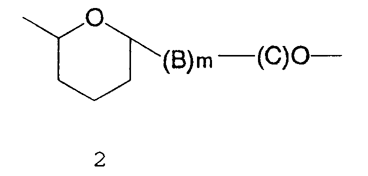 wherein B represents -CH₂-O-CH₂- and m is 0 or 1; Z is a spacer group and T is a carrier moiety, which process comprises:
a) converting a derivative of formula 4: 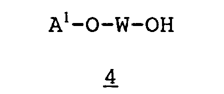 wherein A¹-O- is the residue of an anthracycline bearing at least one primary or secondary hydroxyl group and having the amino group of the sugar moiety protected or replaced by a morpholino group and W is as defined above, into an activated derivative of the formula 5: 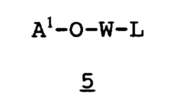 wherein A¹-O- and W are as defined above and L represents an activating group for making an amidic linkage; and
(b)
(i) condensing the resultant compounds of formula 5, as above defined, with a carrier of formula T-NH₂ bearing at least one free amino group, or
(ii) reacting the activated compound of formula 5, with a derivative of formula NH₂-[D]_{d}NH₂, wherein d is an integer of from 0 to 2, [D] represents -NH-CO(CH₂)ₙCO-NH- (n is 2 or 4), optionally deprotecting the resultant derivative of formula 6: 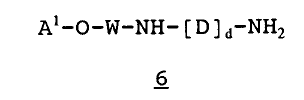 wherein A¹-O-, W, d and [D] are as defined above and condensing it with a compound of formula T-CHO bearing at least one formyl group or T[COOH]ₐ wherein a is as defined above or
(iii) reacting the compound of formula 5 with a 1,4-piperazine and condensing the resultant compound of formula 7: 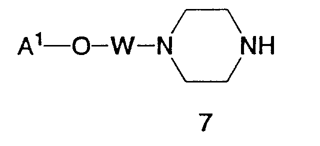 wherein A¹-O- and W are as above defined, with a compound of formula T[COOH]ₐ as defined above, optionally in the presence of a condensing agent to give a conjugate of formula 1, in which the unreacted optionally present activated carboxyl groups may be quenched with a pharmaceutically acceptable amine.

2. A process according to claim 1, wherein the anthracycline bearing at least one primary or secondary hydroxyl group has the formula 3: 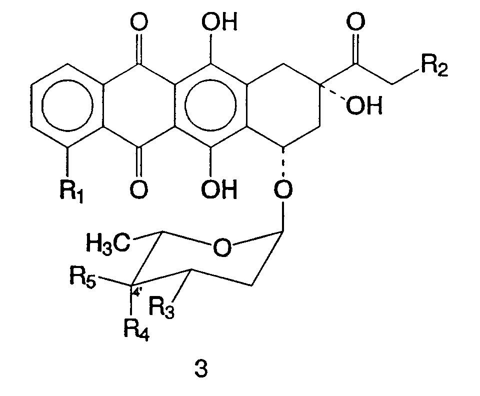 in which:
R₁ is a hydrogen atom, a hydroxy or a methoxy group;
(i) R₂ is a hydroxy group and R₄ or R₅ is a hydrogen atom and the other of R₄ and R₅ is a hydroxy group, R₄ is an iodine atom and R₅ is a hydrogen atom or both R₄ and R₅ are hydrogen atoms or
(ii) R is a hydrogen atom and R₄ or R₅ represents a hydroxy group and the other of R₄ and R₅ is a hydrogen atom;
R₃ is an amino group or represents a nitrogen atom enclosed in a morpholino ring.

3. A process according to claim 2, wherein the moiety A-O- represents: 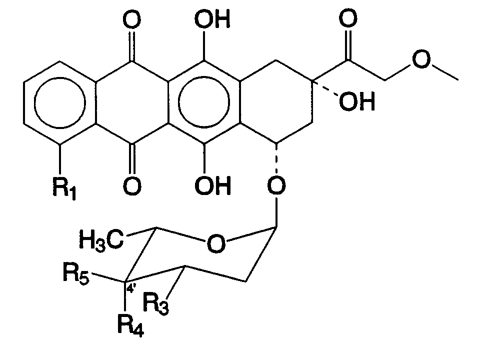 in which R₁ and R₃ are as defined in claim 2 and R₄ or R₅ is a hydrogen atom and the other of R₄ and R₅ is a hydroxy group or R₄ is a hydrogen or iodine atom and R₅ is a hydrogen atom.

4. A process according to claim 2, wherein the moiety A-O- represents: 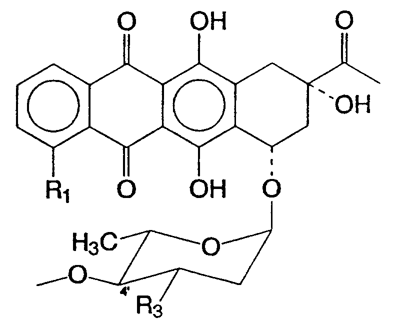 in which R₁ and R₃ are as defined in claim 2.

5. A process according to any one of the preceding claims, wherein the carrier is selected from a polyclonal antibody, or fragment thereof comprising an antigen binding site, capable of binding to a tumor associated antigen; a monoclonal antibody, or fragment thereof comprising an antigen binding site, capable of binding to an antigen preferentially or selectively expressed on tumor cell populations; a peptide or protein capable of preferentially or selectively binding to a tumor cell; and a polymeric carrier.

6. A process according to claim 5, wherein the carrier is selected from an anti-T-cell antibody, an anti-CD5 antibody, an anti-transferrin receptor antibody, an anti-melanoma antibody, an anti-carcinoma marker antibody, an anti-ovarian carcinoma antibody, an anti-breast carcinoma antibody and an anti-bladder carcinoma antibody.

7. A process according to claim 5, wherein the carrier is a growth factor.

8. A process for preparing a pharmaceutical composition comprising admixing a pharmaceutically acceptable carrier or diluent and, as active ingredient, a conjugate which has been prepared by a process as claimed in any one of claims 1 to 7.

9. A process for the preparation of a derivative of formula 4 as claimed in claim 1, which process comprises
(a) condensing an optionally protected anthracycline of the formula A-O-H as defined in claim 1, with the proviso that the amino group of the sugar moiety of the anthracycline A-O-H is in the form of a free or protected amino group, with a dihydropyran carboxylic derivative of formula 8: 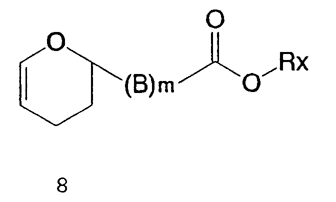 wherein B and m are as defined in claim 1 and Rx is a protecting group;
(b) removing the or each protecting group from the resultant intermediate, thereby to form a derivative of formula 4': 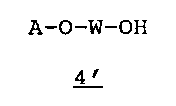 wherein A-O- and W are as defined in claim 1 with the proviso that the amino group of the sugar moiety of the anthracycline residue A-O- is in the form of a free amino group; and
(c)
(i) converting the free amino group of the sugar moiety of the anthracycline of formula 4' into a morpholino derivative, or
(ii) protecting the said free amino group of the said anthracycline of formula 4'.

10. A process according to claim 9 in which the derivative of formula 4 is selected from:
4'-epi-4'-O-(2-carboxytetrahydropyran-6-yl)-3'-deamino-3'-(4-morpholino)-daunorubicin;
14-O-(2-carboxytetrahydropyran-6-yl)-3'-deamino-3'-(2-methoxy-4-morpholino)-doxorubicin; and
14-O-(2-carboxymethyloxymethyl-tetrahydropyran-6-yl)-3'-deamino-3'-(4-morpholino)-doxorubicin.

11. A process for the preparation of a derivative of formula 4' as defined in claim 9, which process comprises:
(a) condensing an optionally protected anthracycline of the formula A-O-H as defined in claim 1, with the proviso that the amino group of the sugar moiety of the anthracycline A-O-H is in the form of a free or protected amino group, with a dihydropyran carboxylic derivative of formula 8 as defined in claim 9; and
(b) removing the or each protecting group from the resultant intermediate.

12. A process according to claim 1 in which the derivative of formula 4' is selected from:
4'-epi-4'-O-(2-carboxytetrahydropyran-6-yl)-daunorubicin;
14-O-(2-carboxytetrahydropyran-6-yl)-doxorubicin; and
14-O-(2-carboxymethyloxymethyl-tetrahydropyran-6-yl)-doxorubicin.

13. A process for the preparation of a derivative of formula 5 as defined in claim 1, which process comprises converting a derivative of formula 4 as defined in claim 1 into a corresponding said derivative of formula 5.

14. A process according to claim 13 in which the derivative of formula 5 is selected from:
4'-epi-4'-O-(2-succinimidocarbonyl-tetrahydropyran-6-yl)-3'-deamino-3'-(4-morpholino)-daunorubicin;
14-O-(2-succinimidocarbonyl-tetrahydropyran-6-yl)-3'-deamino-3'-(2-methoxy-4-morpholino)-doxorubicin; and
14-O-(2-succinimidocarbonylmethyloxymethyl-tetrahydropyran-6-yl)-3'-deamino-3'-(4-morpholino)-doxorubicin.

15. A process for the preparation of a derivative of formula 6 or 7 as defined in claim 1, which process comprises:
(i) reacting a derivative of formula 5 as defined in claim 1 with a derivative of formula NH₂[D]_{d}NH₂ or 1,4-piperazine and, if desired, deprotecting the resultant derivative of formula 6 or 7 thus obtained.

## Claims (Claims for the following Contracting State(s): GR)

1. A process for the preparation of a conjugate of formula 1 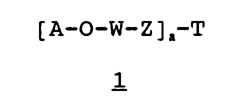 wherein the moiety A-O- is the residue of any anthracycline of formula A-O-H bearing at least one primary or secondary hydroxyl group; a is an integer of from 1 to 30; W is a residue of formula 2 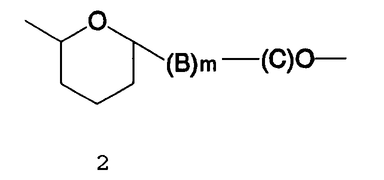 wherein B represents -CH₂-O-CH₂- and m is 0 or 1; Z is a spacer group and T is a carrier moiety which process comprises:
a) converting a derivative of formula 4: 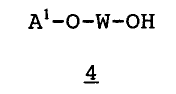 wherein A¹-O- is the residue of an anthracycline bearing at least one primary or secondary hydroxyl group and having the amino group of the sugar moiety protected or replaced by a morpholino group and W is as defined above, into an activated derivative of the formula 5: 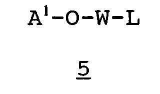 wherein A¹-O- and W are as defined above and L represents an activating group for making an amidic linkage; and
(b)
(i) condensing the resultant compounds of formula 5, as above defined, with a carrier of formula T-NH₂ bearing at least one free amino group or
(ii) reacting the activated compound of formula 5, with a derivative of formula NH₂-[D]_{d}NH₂, wherein d is an integer of from 0 to 2, [D] represents -NH-CO(CH₂)ₙCO-NH- (n is 2 or 4), optionally deprotecting the resultant derivative of formula 6: 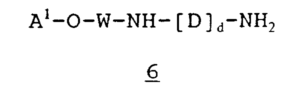 wherein A¹-O-, W, d and [D] are as defined above and condensing it with a compound of formula T-CHO bearing at least one formyl group or T[COOH]ₐ wherein a is as defined above or
(iii) reacting the compound of formula 5 with a 1,4-piperazine and condensing the resultant compound of formula 7: 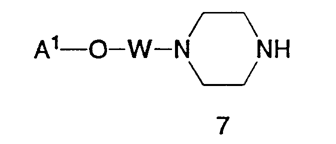 wherein A¹-O- and W are as above defined, with a compound of formula T[COOH]ₐ as defined above, optionally in the presence of a condensing agent to give a conjugate of formula 1, in which the unreacted optionally present activated carboxyl groups may be quenched with a pharmaceutically acceptable amine.

2. A process according to claim 1, wherein the anthracycline bearing at least one primary or secondary hydroxyl group has the formula 3: 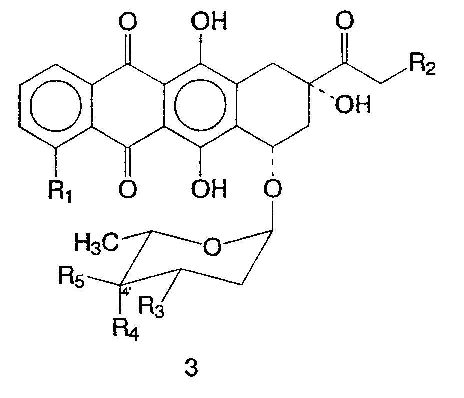 in which:
R₁ is a hydrogen atom, a hydroxy or a methoxy group;
(i) R₂ is a hydroxy group and R₄ or R₅ is a hydrogen atom and the other of R₄ and R₅ is a hydroxy group, R₄ is an iodine atom and R₅ is a hydrogen atom or both R₄ and R₅ are hydrogen atoms or
(ii) R₂ is a hydrogen atom and R₄ or R₅ represents a hydroxy group and the other of R₄ and R₅ is a hydrogen atom;
R₃ is an amino group or represents a nitrogen atom enclosed in a morpholino ring.

3. A process according to claim 2, wherein the moiety A-O- represents: 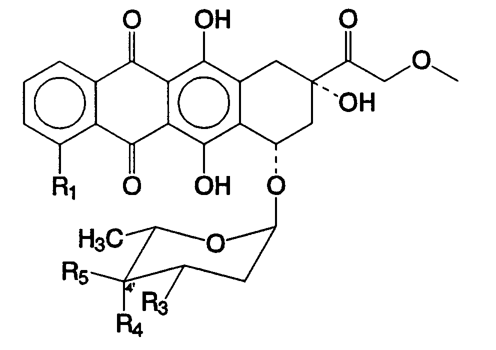 in which R₁ and R₃ are as defined in claim 2 and R₄ or R₅ is a hydrogen atom and the other of R₄ and R₅ is a hydroxy group or R₄ is a hydrogen or iodine atom and R₅ is a hydrogen atom.

4. A conjugate according to claim 2, wherein the moiety A-O- represents: 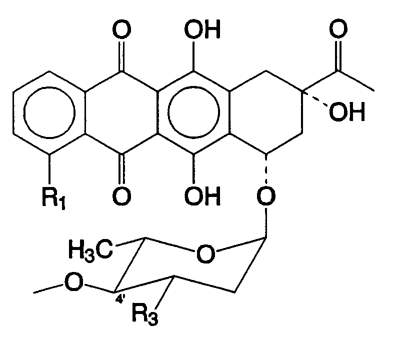 in which R₁ and R₃ are as defined in claim 2.

5. A process according to any one of the preceding claims, wherein the carrier is selected from a polyclonal antibody, or fragment thereof comprising an antigen binding site, capable of binding to a tumor associated antigen; a monoclonal antibody, or fragment thereof comprising an antigen binding site, capable of binding to an antigen preferentially or selectively expressed on tumor cell populations; a peptide or protein capable of preferentially or selectively binding to a tumor cell; and a polymeric carrier.

6. A process according to claim 5, wherein the carrier is selected from an anti-T-cell antibody, an anti-CD5 antibody, an anti-transferrin receptor antibody, an anti-melanoma antibody, an anti-carcinoma marker antibody, an anti-ovarian carcinoma antibody, an anti-breast carcinoma antibody and an anti-bladder carcinoma antibody.

7. A process according to claim 5, wherein the carrier is a growth factor.

8. A process for preparing a pharmaceutical composition comprising admixing a pharmaceutically acceptable carrier or diluent and, as active ingredient, a conjugate which has been prepared by a process as claimed in any one of claims 1 to 7.

9. A derivative of formula 4 as defined in claim 1.

10. A derivative according to claim 9, selected from:
4'-epi-4'-O-(2-carboxytetrahydropyran-6-yl)-3'-deamino-3'-(4-morpholino)-daunorubicin;
14-O-(2-carboxytetrahydropyran-6-yl)-3'-deamino-3'-(2-methoxy-4-morpholino)-doxorubicin; and
14-O-(2-carboxymethyloxymethyl-tetrahydropyran-6-yl)-3'-deamino-3'-(4-morpholino)-doxorubicin.

11. A process for the preparation of a derivative of formula 4 as claimed in claim 9, which process comprises
(a) condensing an optionally protected anthracycline of the formula A-O-H as defined in claim 1, with the proviso that the amino group of the sugar moiety of the anthracycline A-O-H is in the form of a free or protected amino group, with a dihydropyran carboxylic derivative of formula 8: 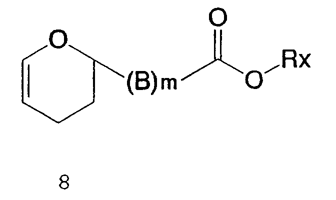 wherein B and m are as defined in claim 1 and Rx is a protecting group;
(b) removing the or each protecting group from the resultant intermediate, thereby to form a derivative of formula 4': 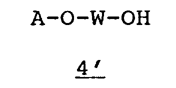 wherein A-O- and W are as defined in claim 1 with the proviso that the amino group of the sugar moiety of the anthracycline residue A-O- is in the form of a free amino group; and
(c)
(i) converting the free amino group of the sugar moiety of the anthracycline of formula 4' into a morpholino derivative, or
(ii) protecting the said free amino group of the said anthracycline of formula 4'.

12. A derivative of formula 4' as defined in claim 11, with the proviso that when the anthracycline is of formula 3 as defined in claim 2 the anthracycline is not linked to the group -O-W-OH through a hydroxy group at R₄.

13. A derivative according to claim 12, selected from:
4'-epi-4'-O-(2-carboxytetrahydropyran-6-yl)-daunorubicin;
14-O-(2-carboxytetrahydropyran-6-yl)-doxorubicin; and
14-O-(2-carboxymethyloxymethyl-tetrahydropyran-6-yl)-doxorubicin.

14. A process for the preparation of a derivative of formula 4' as claimed in claim 12, which process comprises:
(a) condensing an optionally protected anthracycline of the formula A-O-H as defined in claim 1, with the proviso that the amino group of the sugar moiety of the anthracycline A-O-H is in the form of a free or protected amino group, with a dihydropyran carboxylic derivative of formula 8 as defined in claim 11; and
(b) removing the or each protecting group from the resultant intermediate.

15. A derivative of formula 5 as defined in claim 1.

16. A derivative according to claim 15, selected from:
4'-epi-4'-O-(2-succinimidocarbonyl-tetrahydropyran-6-yl)-3'-deamino-3'-(4-morpholino)-daunorubicin;
14-O-(2-succinimidocarbonyl-tetrahydropyran-6-yl)-3'-deamino-3'-(2-methoxy-4-morpholino)-doxorubicin; and
14-O-(2-succinimidocarbonylmethyloxymethyl-tetrahydropyran-6-yl)-3'-deamino-3'-(4-morpholino)-doxorubicin.

17. A process for the preparation of a derivative of formula 5 as claimed in claim 15, which process comprises converting a derivative of formula 4 as defined in claim 1 into a corresponding said derivative of formula 5.

18. A derivative of formula 6 or 7 as defined in claim 1.

19. A process for the preparation of a derivative of formula 6 or 7 as claimed in claim 18, which process comprises:
(i) reacting a derivative of formula 5 as defined in claim 1 with a derivative of formula NH₂[D]_{d}NH₂ or 1,4-piperazine and, if desired, deprotecting the resultant derivative of formula 6 or 7 thus obtained.

20. A compound of formula: 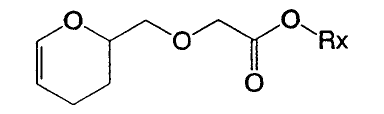 where Rₓ is a protecting group.

21. A derivative according to claim 20, selected from:
2-ethoxycarbonylmethyloxymethyl-3,4-dihydro-2H-pyrane; and
2-methoxycarbonylmethyloxymethyl-3,4-dihydro-2H-pyrane.

## Patentansprüche (Patentansprüche für folgende(n) Vertragsstaat(en): AT, BE, CH, DE, DK, FR, GB, IT, LI, NL, SE)

1. Konjugat der Formel (1)
(A-O-W-Z)ₐ-T (1)
worin der A-O-Anteil der Rest jedes Anthracyclins der Formel A-O-H ist, der zumindest eine primäre oder sekundäre Hydroxylgruppe trägt; a eine ganze Zahl zwischen 1 und 3 ist; W ein Rest der Formel (2) ist 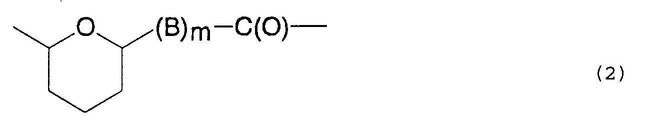 worin B -CH₂-O-CH₂- darstellt und m 0 oder 1 ist; Z eine Spacergruppe ist und T ein Trägeranteil ist.

2. Konjugat gemäss Anspruch 1, worin:
(i) Z -NH- darstellt und T-Z sich von einem Träger der Formel T-NH₂ ableitet, der zumindest eine freie Aminogruppe trägt, oder
(ii) Z NH-(D)_{d}-N=CH ist, worin d eine ganze Zahl zwischen 0 und 20 ist, (D) -NH-CO(CH₂)ₙCO-NH- darstellt (n ist 2 oder 4) und T-Z sich von einem Träger der Formel T-CHO ableitet, der zumindest eine Formylgruppe oder T(COOH)ₐ trägt, worin a wie oben definiert ist, oder
(iii) Z ein Piperazinylcarbonylanteil oder eine Gruppe der Formel -NH(D)_{d}-NH-CO- ist, worin (D) und d wie oben definiert sind und T-Z sich von einem Träger der Formel T-(COOH)ₐ ableitet, wie oben definiert.

3. Konjugat gemäss Anspruch 1, worin das Anthracyclin, das zumindest eine primäre oder sekundäre Hydroxylgruppe trägt, die Formel (3) hat: 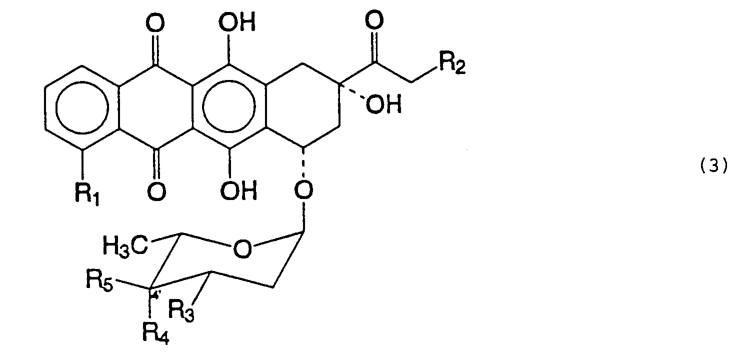 in der
R₁ ein Wasserstoffatom, eine Hydroxy- oder Methoxygruppe ist;
(i) R₂ eine Hydroxygruppe ist und eine von R₄ oder R₅ ein Wasserstoffatom und die andere eine Hydroxygruppe ist, R₄ ein Iodatom und R₅ ein Wasserstoffatom ist, oder R₄ und R₅ Wasserstoffatome sind; oder
(ii) R₂ ist ein Wasserstoffatom und eine von R₄ oder R₅ stellt eine Hydroxygruppe dar und die andere ist ein Wasserstoffatom;
R₃ ist eine Aminogruppe oder stellt ein Stickstoffatom, eingeschlossen in einem Morpholinring, dar.

4. Konjugat gemäss Anspruch 3, worin der A-O-Anteil darstellt: 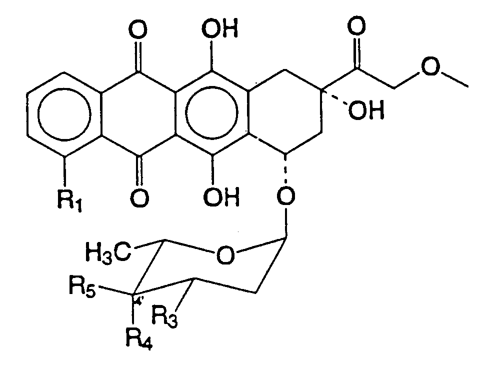 in dem R₁ und R₃ wie in Anspruch 3 definiert sind, und eine von R₄ oder R₅ ist ein Wasserstoffatom und die andere stellt eine Hydroxygruppe dar oder R₄ ist ein Wasserstoff- oder Iodatom und R₅ ist ein Wasserstoffatom.

5. Konjugat gemäss Anspruch 3, worin der A-O-Anteil darstellt: 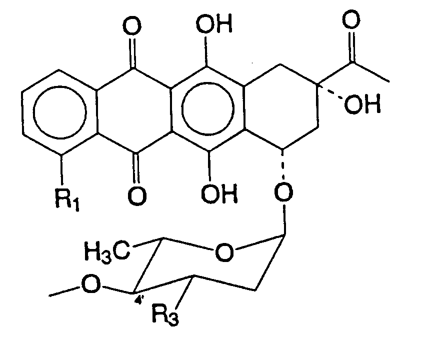 worin R₁ und R₃ wie in Anspruch 2 definiert sind.

6. Konjugat gemäss einem der vorstehenden Ansprüche, worin der Träger ausgewählt ist aus einem polyklonalen Antikörper oder einem Fragment davon, welche eine Antigen-Bindungsstelle enthalten, die an ein Tumor-assoziiertes Antigen binden kann; einem monoklonalen Antikörper oder einem Fragment davon, welche eine Antigen-Bindungsstelle enthalten, die an ein Antigen, das bevorzugt oder selektiv auf Tumorzellpopulationen exprimiert ist, binden kann; einem Peptid oder Protein, das bevorzugt oder selektiv an eine Tumorzelle binden kann; und einem polymeren Träger.

7. Konjugat gemäss Anspruch 1, worin der Träger von einem Anti-T-Zellen-Antikörper, einem Anti-CD5-Antikörper, einem Anti-Transferrinrezeptor-Antikörper, einem Anti-Melanoma-Antikörper, einem Anti-Karzinommarker-Antikörper, einem Anti-Eierstockkarzinom-Antikörper, einem Anti-Brustkarzinom-Antikörper und einem Anti-Blasenkarzinom-Antikörper ausgewählt ist.

8. Konjugat gemäss Anspruch 6, worin der Träger ein Wachstumsfaktor ist.

9. Verfahren zur Herstellung eines Konjugats der Formel (1), wie in Anspruch 1 definiert, umfassend:
(a) Umwandlung eines Derivats der Formel (4):
A¹-O-W-OH (4)
worin A¹-O- der Rest eines Anthracyclins ist, das zumindest eine primäre oder sekundäre Hydroxylgruppe trägt und die Aminogruppe des Zuckeranteils geschützt oder durch eine Morpholinogruppe ersetzt ist, und W wie in Anspruch 1 definiert ist, in ein aktiviertes Derivat der Formel (5)
A¹-O-W-L (5)
worin A¹-O- und W wie oben definiert sind und L eine aktivierte Gruppe darstellt, die die Amidbindung herbeiführt; und
(b)
(i) Kondensieren der resultierenden Verbindungen der Formel (5), wie oben definiert, mit einer Verbindung der Formel T-NH₂, wie in Anspruch 2 definiert, oder
(ii) Umsetzung der aktivierten Verbindung der Formel (5) mit einem Derivat der Formel NH₂-(D)_{d}NH₂, worin (D) und d wie in Anspruch 2 definiert sind, wobei gegebenenfalls am resultierenden Derivat der Formel (6)
A¹-O-W-NH-(D)_{d}-NH₂ (6)
worin A¹-O-, W, d und (D) wie in Anspruch 2 definiert sind, die Schutzgruppe abgespalten wird, und Kondensieren mit einer Verbindung der Formel T-CHO oder T-(COOH)ₐ, wie in Anspruch 2 definiert, oder
(iii) Umsetzung der Verbindung der Formel (5) mit 1,4-Piperazin und Kondensieren der resultierenden Verbindung der Formel (7) 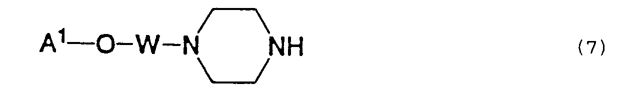 worin A¹-O- und W wie oben definiert sind, mit einer Verbindung der Formel T(COOH)ₐ, wie oben definiert, gegebenenfalls in Anwesenheit eines Kondensierungsmittels, um ein Konjugat der Formel (1) zu bilden, in dem die freien, gegebenenfalls anwesenden aktivierten Carboxylgruppen mit einem pharmazeutisch akzeptablen Amin umgesetzt sein können.

10. Pharmazeutische Zusammensetzung, umfassend einen pharmazeutisch akzeptablen Träger oder ein Verdünnungsmittel, und als aktiven Bestandteil ein Konjugat gemäss einem der Ansprüche 1 bis 8, oder hergestellt durch das Verfahren gemäss Anspruch 9.

11. Derivat der Formel (4), wie in Anspruch 9 definiert.

12. Derivat gemäss Anspruch 11, ausgewählt aus:
4'-Epi-4'-O-(2-carboxytetrahydropyran-6-yl)-3'-desamino-3'-(4-morpholino) -daunorubicin;
14-O-(2-Carboxytetrahydropyran-6-yl)-3'-desamino-3'-(2-methoxy-4-morpholino)-doxorubicin; und
14-O-(2-Carboxymethyloxymethyl-tetrahydropyran-6-yl)-3'-desamino-3'-(4-morpholino)-doxorubicin.

13. Verfahren zur Herstellung eines Derivats der Formel (4) gemäss Anspruch 11, umfassend:
(a) Kondensieren eines gegebenenfalls geschützten Anthracyclins der Formel A-O-H, wie in Anspruch 1 definiert, unter der Voraussetzung, dass die Aminogruppe des Zuckeranteils des Anthracyclins A-O-H in Form einer freien oder geschützten Aminogruppe vorliegt, mit einem Dihydropyrancarbonsäurederivat der Formel (8) : 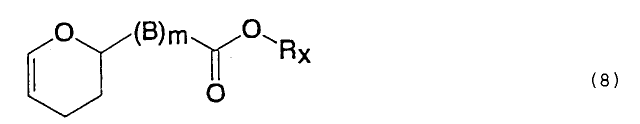 worin B und m wie in Anspruch 1 definiert sind und Rₓ eine Schutzgruppe ist;
(b) Entfernen der oder jeder Schutzgruppe von dem resultierenden Zwischenprodukt zur Bildung eines Derivats der Formel (4')
A-O-W-OH (4')
worin A-O- und W wie in Anspruch 1 definiert sind, unter der Voraussetzung, dass die Aminogruppe der Zuckerkomponente des Anthracyclinrests A-O- in Form einer freien Aminogruppe vorliegt; und
(c)
(i) Umwandlung der freien Aminogruppe der Zuckerkomponente des Anthracyclins der Formel (4') in ein Morpholinoderivat, oder
(ii) Schützen der freien Aminogruppe des Anthracyclins der Formel (4') .

14. Derivat der Formel (4'), wie in Anspruch 13 definiert, unter der Voraussetzung, dass, wenn das Anthracyclin eines der Formel (3) gemäss Anspruch 3 ist, das Anthracyclin nicht an eine Gruppe -O-W-OH durch eine Hydroxylgruppe an R₄ gebunden ist.

15. Derivat gemäss Anspruch 14, ausgewählt aus
4'-Epi-4'-O-(2-carboxytetrahydropyran-6-yl)-daunorubicin;
14-O-(2-Carboxytetrahydropyran-6-yl)-doxorubicin; und
14-O-(2-Carboxymethyloxymethyl-tetrahydropyran-6-yl)-doxorubicin.

16. Verfahren zur Herstellung eines Derivats der Formel (4') gemäss Anspruch 14, umfassend:
(a) Kondensieren eines gegebenenfalls geschützten Anthracyclins der Formel A-O-H, wie in Anspruch 1 definiert, unter der Voraussetzung, dass die Aminogruppe des Zuckeranteils des Anthracyclins A-O-H in Form einer freien oder geschützten Aminogruppe vorliegt, mit einem Dihydropyrancarbonsäurederivat der Formel (8), wie in Anspruch 13 definiert; und
(b) Entfernen der oder jeder Schutzgruppe des resultierenden Zwischenprodukts.

17. Derivat der Formel 5, wie in Anspruch 9 definiert.

18. Derivat gemäss Anspruch 17, ausgewählt aus:
4'-Epi-4'-O-(2-succinimidocarbonyl-tetrahydropyran-6-yl)-3'-desamino-3'-(4-morpholino)-daunorubicin;
14-O-(2-Succinimidocarbonyl-tetrahydropyran-6-yl)-3'-desamino-3'-(2-methoxy-4-morpholino)-doxorubicin; und
14-O-(2-Succinimidocarbonylmethyloxymethyl-tetrahydropyran-6-yl)-3'-desamino-3'-(4-morpholino)-doxorubicin.

19. Verfahren zur Herstellung eines Derivats der Formel (5) gemäss Anspruch 17, umfassend die Umwandlung eines Derivats der Formel (4), wie in Anspruch 9 definiert, in ein korrespondierendes Derivat der Formel (5).

20. Derivat der Formel (6) oder (7) gemäss Anspruch 9.

21. Verfahren zur Herstellung eines Derivats der Formel (6) oder (7) gemäss Anspruch 20, umfassend:
(i) Umsetzen eines Derivats der Formel (5), wie in Anspruch 9 definiert, mit einem Derivat der Formel (NH₂(D)_{d}NH₂ oder 1,4-Piperazin und, falls gewünscht, Entfernen der Schutzgruppe des auf diese Weise erhaltenen Derivats der Formel (6) oder (7).

22. Verbindung der Formel 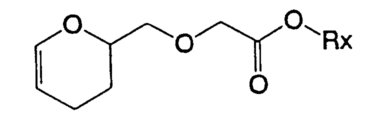 worin Rₓ eine Schutzgruppe ist.

23. Derivat gemäss Anspruch 22, ausgewählt aus:
2-Ethoxycarbonylmethyloxymethyl-3,4-dihydro-2H-pyran; und
2-Methoxycarbonylmethyloxymethyl-3,4-dihydro-2H-pyran.

24. Konjugat der Formel 1 gemäss Anspruch 1 für die Verwendung als Anti-Tumormittel.

## Patentansprüche (Patentansprüche für folgende(n) Vertragsstaat(en): ES)

1. Verfahren zur Herstellung eines Konjugats der Formel (1)
(A-O-W-Z)ₐ-T (1)
worin der A-O-Anteil der Rest jedes Anthracyclins der Formel A-O-H ist, der zumindest eine primäre oder sekundäre Hydroxylgruppe trägt; a ist eine ganze Zahl zwischen 1 und 30; W ist ein Rest der Formel (2) 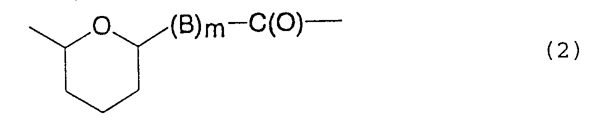 worin B -CH₂-O-CH₂- darstellt und m ist 0 oder 1; Z ist eine Spacergruppe und T ist ein Trägeranteil, wobei das Verfahren umfasst:
(a) Umwandeln eines Derivats der Formel (4)
A¹-O-W-OH (4)
worin A¹-O- der Rest eines Anthracyclins ist, das mindestens eine primäre oder sekundäre Hydroxylgruppe trägt, wobei die Aminogruppe des Zuckeranteils geschützt oder durch eine Morpholingruppe ersetzt ist, und W wie oben definiert ist, in ein aktiviertes Derivat der Formel (5)
A¹-O-W-L (5)
worin A¹-O- und W wie oben definiert sind und L eine Aktivierungsgruppe zur Ausbildung einer Amidbrücke bedeutet; und
(b)
(i) Kondensieren der erhaltenen Verbindungen der Formel (5), wie oben definiert, mit einem Träger der Formel T-NH₂, der mindestens eine freie Aminogruppe trägt, oder
(ii) Umsetzen der aktivierten Verbindung der Formel (5) mit einem Derivat der Formel NH₂-(D)_{d}NH₂, worin d eine ganze Zahl von 0 bis 2 ist, (D) bedeutet -NH-CO(CH₂)ₙCO-NH- (n ist 2 oder 4), wahlweise Entfernen der Schutzgruppe des erhaltenen Derivats der Formel (6)
A¹-O-W-NH-(D)_{d}-NH₂ (6)
worin A¹-O-, W, d und (D) wie oben definiert sind, und Kondensieren mit einer Verbindung der Formel T-CHO, die mindestens eine Formylgruppe oder T(COOH)ₐ trägt, worin a wie oben definiert ist, oder
(iii) Umsetzen der Verbindung der Formel (5) mit 1,4-Piperazin und Kondensieren der erhaltenen Verbindung der Formel (7) 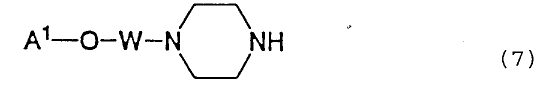 worin A¹-O- und W wie oben definiert sind, mit einer Verbindung der Formel T(COOH)ₐ, wie oben definiert, wahlweise in Gegenwart eines Kondensierungsmittels, unter Erhalt eines Konjugats mit der Formel (1), bei der die nicht-umgesetzte, wahlweise vorhandene aktivierte Carboxylgruppe mit einem pharmazeutisch verträglichen Amin gequencht werden kann.

2. Verfahren nach Anspruch 1, worin das Anthracyclin, das zumindest eine primäre oder sekundäre Hydroxylgruppe trägt, die Formel (3) hat: 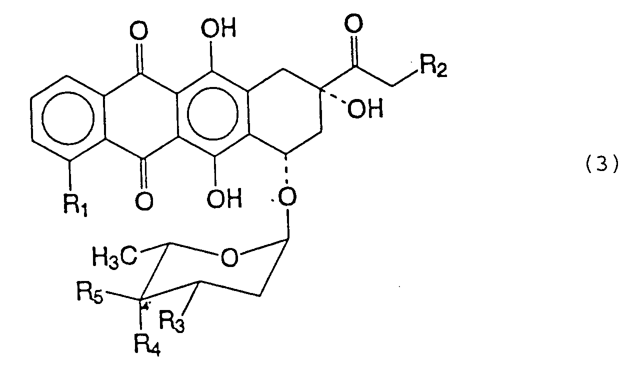 in der
R₁ ein Wasserstoffatom, eine Hydroxy- oder Methoxygruppe ist;
(i) R₂ ist eine Hydroxygruppe und eine von R₄ oder R₅ ist ein Wasserstoffatom und die andere ist eine Hydroxygruppe, R₄ ist ein Iodatom und R₅ ist ein Wasserstoffatom, oder R₄ und R₅ sind Wasserstoffatome; oder
(ii) R₂ ist ein Wasserstoffatom und eine von R₄ oder R₅ stellt eine Hydroxygruppe dar und die andere ist ein Wasserstoffatom;
R₃ ist eine Aminogruppe oder stellt ein Stickstoffatom, eingeschlossen in einem Morpholinring, dar.

3. Verfahren nach Anspruch 2, worin der A-O-Anteil darstellt: 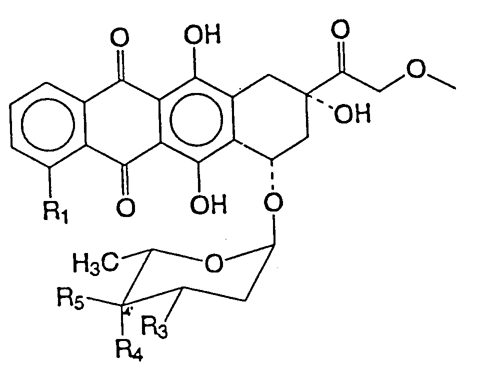 in dem R₁ und R₃ wie in Anspruch 2 definiert sind, und eine von R₄ oder R₅ ist ein Wasserstoffatom und die andere stellt eine Hydroxygruppe dar oder R₄ ist ein Wasserstoff- oder Iodatom und R₅ ist ein Wasserstoffatom.

4. Verfahren nach Anspruch 2, worin der A-O-Anteil darstellt: 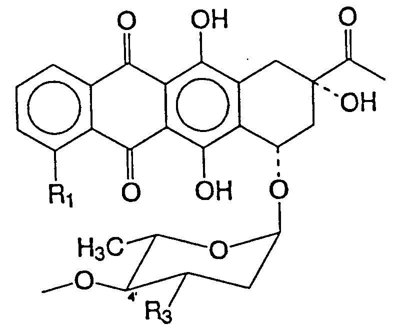 worin R₁ und R₃ wie in Anspruch 2 definiert sind.

5. Verfahren nach einem der vorstehenden Ansprüche, worin der Träger ausgewählt ist aus einem polyklonalen Antikörper oder einem Fragment davon, welche eine Antigen-Bindungsstelle enthalten, die an ein Tumor-assoziiertes Antigen binden kann; einem monoklonalen Antikörper oder einem Fragment davon, welche eine Antigen-Bindungsstelle enthalten, die an ein Antigen, das bevorzugt oder selektiv auf Tumorzellpopulationen exprimiert ist, binden kann; einem Peptid oder Protein, das bevorzugt oder selektiv an eine Tumorzelle binden kann; und einem polymeren Träger.

6. Verfahren nach Anspruch 5, worin der Träger von einem Anti-T-Zellen-Antikörper, einem Anti-CD5-Antikörper, einem Anti-Transferrinrezeptor-Antikörper, einem Anti-Melanoma-Antikörper, einem Anti-Karzinommarker-Antikörper, einem Anti-Eierstockkarzinom-Antikörper, einem Anti-Brustkarzinom-Antikörper und einem Anti-Blasenkarzinom-Antikörper ausgewählt ist.

7. Verfahren nach Anspruch 5, worin der Träger ein Wachstumsfaktor ist.

8. Verfahren zur Herstellung einer pharmazeutischen Zusammensetzung, umfassend das Vermischen eines pharmazeutisch verträglichen Trägers und/oder Verdünners und als Wirkstoff eines Konjugats, hergestellt durch ein Verfahren nach einem der Ansprüche 1 bis 7.

9. Verfahren zur Herstellung eines Derivats der Formel (4) gemäss Anspruch 1, umfassend:
(a) Kondensieren eines gegebenenfalls geschützten Anthracyclins der Formel A-O-H, wie in Anspruch 1 definiert, unter der Voraussetzung, dass die Aminogruppe des Zuckeranteils des Anthracyclins A-O-H in Form einer freien oder geschützten Aminogruppe vorliegt, mit einem Dihydropyrancarbonsäurederivat der Formel (8) : 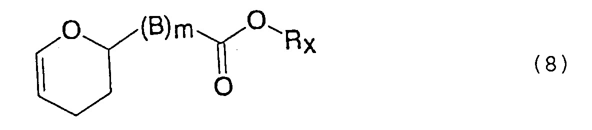 worin B und m wie in Anspruch 1 definiert sind und Rₓ eine Schutzgruppe ist;
(b) Entfernen der oder jeder Schutzgruppe von dem resultierenden Zwischenprodukt zur Bildung eines Derivats der Formel (4')
A-O-W-OH (4')
worin A-O- und W wie in Anspruch 1 definiert sind, unter der Voraussetzung, dass die Aminogruppe der Zuckerkomponente des Anthracyclinrests A-O- in Form einer freien Aminogruppe vorliegt; und
(c)
(i) Umwandlung der freien Aminogruppe der Zuckerkomponente des Anthracyclins der Formel (4') in ein Morpholinoderivat, oder
(ii) Schützen der freien Aminogruppe des Anthracyclins der Formel (4') .

10. Verfahren nach Anspruch 9, ausgewählt aus:
4'-Epi-4'-O-(2-carboxytetrahydropyran-6-yl)-3'-desamino-3'-(4-morpholino)-daunorubicin;
14-O-(2-Carboxytetrahydropyran-6-yl)-3'-desamino-3'-(2-methoxy-4-morpholino)-doxorubicin; und
14-O-(2-Carboxymethyloxymethyl-tetrahydropyran-6-yl)-3'-desamino-3'-(4-morpholino)-doxorubicin.

11. Verfahren zur Herstellung eines Derivats der Formel (4') nach Anspruch 9, umfassend:
(a) Kondensieren eines gegebenenfalls geschützten Anthracyclins der Formel A-O-H, wie in Anspruch 1 definiert, unter der Voraussetzung, dass die Aminogruppe des Zuckeranteils des Anthracyclins A-O-H in Form einer freien oder geschützten Aminogruppe vorliegt, mit einem Dihydropyrancarbonsäurederivat der Formel (8), wie in Anspruch 9 definiert; und
(b) Entfernen der oder jeder Schutzgruppe des resultierenden Zwischenprodukts.

12. Verfahren nach Anspruch 1, wobei das Derivat der Formel (4') ausgewählt aus
4'-Epi-4'-O-(2-carboxytetrahydropyran-6-yl)-daunorubicin;
14-O-(2-Carboxytetrahydropyran-6-yl)-doxorubicin; und
14-O-(2-Carboxymethyloxymethyl-tetrahydropyran-6-yl)-doxorubicin.

13. Verfahren zur Herstellung eines Derivats der Formel (5) nach Anspruch 1, umfassend die Umwandlung eines Derivats der Formel (4), wie in Anspruch 1 definiert, in ein korrespondierendes Derivat der Formel (5).

14. Verfahren nach Anspruch 13, wobei das Derivat der Formel (5) ausgewählt ist aus:
4'-Epi-4'-O-(2-succinimidocarbonyl-tetrahydropyran-6-yl)-3'-desamino-3'-(4-morpholino)-daunorubicin;
14-O-(2-Succinimidocarbonyl-tetrahydropyran-6-yl)-3'-desamino-3'-(2-methoxy-4-morpholino)-doxorubicin; und
14-O-(2-Succinimidocarbonylmethyloxymethyl-tetrahydropyran-6-yl)-3'-desamino-3'-(4-morpholino)-doxorubicin.

15. Verfahren zur Herstellung eines Derivats der Formel (6) oder (7) nach Anspruch 1, umfassend:
(i) Umsetzen eines Derivats der Formel (5), wie in Anspruch 1 definiert, mit einem Derivat der Formel NH₂(D)_{d}NH₂ oder 1,4-Piperazin und, falls gewünscht, Entfernen der Schutzgruppe des auf diese Weise erhaltenen Derivats der Formel (6) oder (7).

## Patentansprüche (Patentansprüche für folgende(n) Vertragsstaat(en): GR)

1. Verfahren zur Herstellung eines Konjugats der Formel (1)
(A-O-W-Z)ₐ-T (1)
worin der A-O-Anteil der Rest jedes Anthracyclins der Formel A-O-H ist, der zumindest eine primäre oder sekundäre Hydroxylgruppe trägt; a ist eine ganze Zahl zwischen 1 und 30; W ist ein Rest der Formel (2) 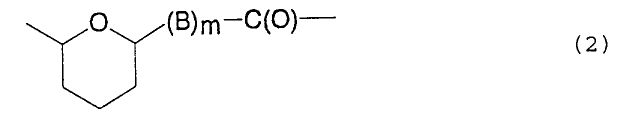 worin B -CH₂-O-CH₂- darstellt und m ist 0 oder 1; Z ist eine Spacergruppe und T ist ein Trägeranteil, wobei das Verfahren umfasst:
(a) Umwandeln eines Derivats der Formel (4)
A¹-O-W-OH (4)
worin A¹-O- der Rest eines Anthracyclins ist, das mindestens eine primäre oder sekundäre Hydroxylgruppe trägt, wobei die Aminogruppe des Zuckeranteils geschützt oder durch eine Morpholingruppe ersetzt ist, und W wie oben definiert ist, in ein aktiviertes Derivat der Formel (5)
A¹-O-W-L (5)
worin A¹-O- und W wie oben definiert sind und L eine Aktivierungsgruppe zur Ausbildung einer Amidbrücke bedeutet; und
(b)
(i) Kondensieren der erhaltenen Verbindungen der Formel (5), wie oben definiert, mit einem Träger der Formel T-NH₂, der mindestens eine freie Aminogruppe trägt, oder
(ii) Umsetzen der aktivierten Verbindung der Formel (5) mit einem Derivat der Formel NH₂-(D)_{d}NH₂, worin d eine ganze Zahl von 0 bis 2 ist, (D) bedeutet -NH-CO(CH₂)ₙCO-NH- (n ist 2 oder 4), wahlweise Entfernen der Schutzgruppe des erhaltenen Derivats der Formel (6)
A¹-O-W-NH-(D)_{d}-NH₂ (6)
worin A¹-O-, W, d und (D) wie oben definiert sind, und Kondensieren mit einer Verbindung der Formel T-CHO, die mindestens eine Formylgruppe oder T(COOH)ₐ trägt, worin a wie oben definiert ist, oder
(iii) Umsetzen der Verbindung der Formel (5) mit 1,4-Piperazin und Kondensieren der erhaltenen Verbindung der Formel (7) 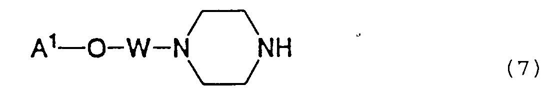 worin A¹-O- und W wie oben definiert sind, mit einer Verbindung der Formel T(COOH)ₐ, wie oben definiert, wahlweise in Gegenwart eines Kondensierungsmittels, unter Erhalt eines Konjugats mit der Formel (1), bei der die nicht-umgesetzte, wahlweise vorhandene aktivierte Carboxylgruppe mit einem pharmazeutisch verträglichen Amin gequencht werden kann.

2. Verfahren nach Anspruch 1, worin das Anthracyclin, das zumindest eine primäre oder sekundäre Hydroxylgruppe trägt, die Formel (3) hat: 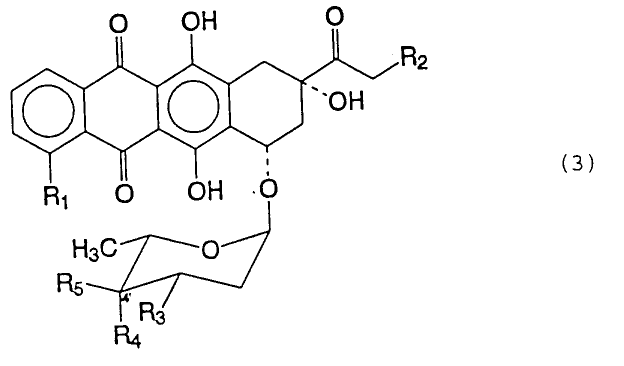 in der
R₁ ein Wasserstoffatom, eine Hydroxy- oder Methoxygruppe ist;
(i) R₂ eine Hydroxygruppe ist und eine von R₄ oder R₅ ein Wasserstoffatom und die andere eine Hydroxygruppe ist, R₄ ein Iodatom und R₅ ein Wasserstoffatom ist, oder R₄ und R₅ Wasserstoffatome sind; oder
(ii) R₂ ist ein Wasserstoffatom und eine von R₄ oder R₅ stellt eine Hydroxygruppe dar und die andere ist ein Wasserstoffatom;
R₃ ist eine Aminogruppe oder stellt ein Stickstoffatom, eingeschlossen in einem Morpholinring, dar.

3. Verfahren nach Anspruch 2, worin der A-O-Anteil darstellt: 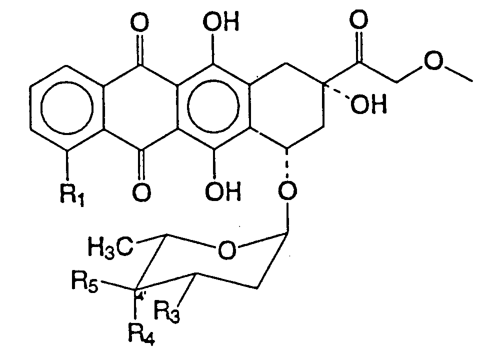 in dem R₁ und R₃ wie in Anspruch 2 definiert sind, und eine von R₄ oder R₅ ist ein Wasserstoffatom und die andere stellt eine Hydroxygruppe dar oder R₄ ist ein Wasserstoff- oder Iodatom und R₅ ist ein Wasserstoffatom.

4. Verfahren nach Anspruch 2, worin der A-O-Anteil darstellt: 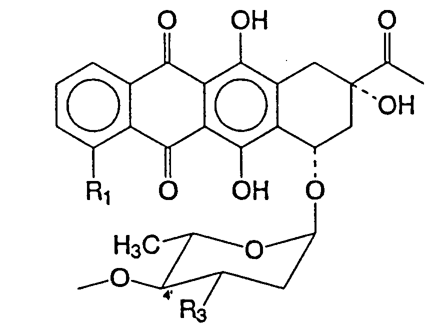 worin R₁ und R₃ wie in Anspruch 2 definiert sind.

5. Verfahren nach einem der vorhergehenden Ansprüche, worin der Träger ausgewählt ist aus einem polyklonalen Antikörper oder einem Fragment davon, welche eine Antigen-Bindungsstelle enthalten, die an ein Tumor-assoziiertes Antigen binden kann; einem monoklonalen Antikörper oder einem Fragment davon, welche eine Antigen-Bindungsstelle enthalten, die an ein Antigen, das bevorzugt oder selektiv auf Tumorzellpopulationen exprimiert ist, binden kann; einem Peptid oder Protein, das bevorzugt oder selektiv an eine Tumorzelle binden kann; und einem polymeren Träger.

6. Verfahren nach Anspruch 5, worin der Träger von einem Anti-T-Zellen-Antikörper, einem Anti-CD5-Antikörper, einem Anti-Transferrinrezeptor-Antikörper, einem Anti-Melanoma-Antikörper, einem Anti-Karzinommarker-Antikörper, einem Anti-Eierstockkarzinom-Antikörper, einem Anti-Brustkarzinom-Antikörper und einem Anti-Blasenkarzinom-Antikörper ausgewählt ist.

7. Verfahren nach Anspruch 5, worin der Träger ein Wachstumsfaktor ist.

8. Verfahren zur Herstellung einer pharmazeutischen Zusammensetzung, umfassend das Vermischen eines pharmazeutisch verträglichen Trägers und/oder Verdünners und als Wirkstoff eines Konjugats, hergestellt durch ein Verfahren nach einem der Ansprüche 1 bis 7.

9. Derivat der Formel (4), wie in Anspruch 1 definiert.

10. Verfahren nach Anspruch 9, in dem das Derivat ausgewählt ist aus:
4'-Epi-4'-O-(2-carboxytetrahydropyran-6-yl)-3'-desamino-3'-(4-morpholino)-daunorubicin;
14-O-(2-Carboxytetrahydropyran-6-yl)-3'-desamino-3'-(2-methoxy-4-morpholino)-doxorubicin; und
14-O-(2-Carboxymethyloxymethyl-tetrahydropyran-6-yl)-3'-desamino-3'-(4-morpholino)-doxorubicin.

11. Verfahren zur Herstellung eines Derivats der Formel (4) gemäss Anspruch 1, wobei das Verfahren umfasst:
(a) Kondensieren eines gegebenenfalls geschützten Anthracyclins der Formel A-O-H, wie in Anspruch 1 definiert, unter der Voraussetzung, dass die Aminogruppe des Zuckeranteils des Anthracyclins A-O-H in Form einer freien oder geschützten Aminogruppe vorliegt, mit einem Dihydropyrancarbonsäurederivat der Formel (8): 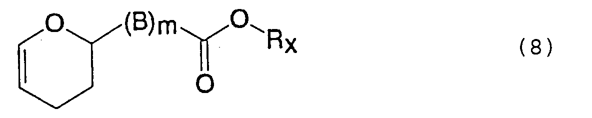 worin B und m wie in Anspruch 1 definiert sind und Rₓ eine Schutzgruppe ist;
(b) Entfernen der oder jeder Schutzgruppe von dem resultierenden Zwischenprodukt zur Bildung eines Derivats der Formel (4')
A-O-W-OH (4')
worin A-O- und W wie in Anspruch 1 definiert sind, unter der Voraussetzung, dass die Aminogruppe der Zuckerkomponente des Anthracyclinrests A-O- in Form einer freien Aminogruppe vorliegt; und
(c)
(i) Umwandlung der freien Aminogruppe der Zuckerkomponente des Anthracyclins der Formel (4') in ein Morpholinoderivat, oder
(ii) Schützen der freien Aminogruppe des Anthracyclins der Formel (4').

12. Derivat der Formel (4') nach Anspruch 11, unter der Voraussetzung, dass, wenn das Anthracyclin eines der Formel (3) gemäss Anspruch 2 ist, das Anthracyclin nicht an eine Gruppe -O-W-OH durch eine Hydroxylgruppe an R₄ gebunden ist.

13. Derivat gemäss Anspruch 12, ausgewählt aus
4'-Epi-4'-O-(2-carboxytetrahydropyran-6-yl)-daunorubicin;
14-O-(2-Carboxytetrahydropyran-6-yl)-doxorubicin; und
14-O-(2-Carboxymethyloxymethyl-tetrahydropyran-6-yl)-doxorubicin.

14. Verfahren zur Herstellung eines Derivats der Formel (4') nach Anspruch 12, wobei das Verfahren umfasst:
(a) Kondensieren eines gegebenenfalls geschützten Anthracyclins der Formel A-O-H, wie in Anspruch 1 definiert, unter der Voraussetzung, dass die Aminogruppe des Zuckeranteils des Anthracyclins A-O-H in Form einer freien oder geschützten Aminogruppe vorleigt, mit einem Dihydropyrancarbonsäurederivat der Formel (8), wie in Anspruch 11 definiert; und
(b) Entfernen der oder jeder Schutzgruppe des resultierenden Zwischenprodukts.

15. Derivat der Formel 5, wie in Anspruch 1 definiert.

16. Derivat gemäss Anspruch 15, ausgewählt aus:
4'-Epi-4'-O-(2-succinimidocarbonyl-tetrahydropyran-6-yl)-3'-desamino-3'-(4-morpholino)-daunorubicin;
14-O-(2-Succinimidocarbonyl-tetrahydropyran-6-yl)-3'-desamino-3'-(2-methoxy-4-morpholino)-doxorubicin; und
14-O-(2-Succinimidocarbonylmethyloxymethyl-tetrahydropyran-6-yl)-3'-desamino-3'-(4-morpholino)-doxorubicin.

17. Verfahren zur Herstellung eines Derivats der Formel (5) gemäss Anspruch 15, umfassend die Umwandlung eines Derivats der Formel (4), wie in Anspruch 1 definiert, in ein korrespondierendes Derivat der Formel (5).

18. Derivat der Formel (6) oder (7) gemäss Anspruch 1.

19. Verfahren zur Herstellung eines Derivats der Formel (6) oder (7) gemäss Anspruch 18, umfassend:
(i) Umsetzen eines Derivats der Formel (5), wie in Anspruch 1 definiert, mit einem Derivat der Formel NH₂(D)_{d}NH₂ oder 1,4-Piperazin und, falls gewünscht, Entfernen der Schutzgruppe des auf diese Weise erhaltenen Derivats der Formel (6) oder (7).

20. Verbindung der Formel 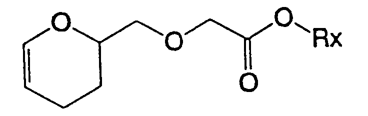 worin Rₓ eine Schutzgruppe ist.

21. Derivat nach Anspruch 20, ausgewählt aus:
2-Ethoxycarbonylmethyloxymethyl-3,4-dihydro-2H-pyran;
und
2-Methoxycarbonylmethyloxymetnyl-3,4-dihydro-2H-pyran.

## Revendications (Revendications pour l'(les) Etat(s) contractant(s) suivant(s): AT, BE, CH, DE, DK, FR, GB, IT, LI, NL, SE)

1. Conjugué de formule 1 : 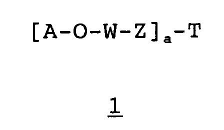 dans laquelle :
- la fraction A-O- est le reste de n'importe quelle anthracycline de formule A-O-H portant au moins un groupe hydroxyle primaire ou secondaire ;
- a est un entier de 1 à 30 ;
- W est un reste de formule 2 : 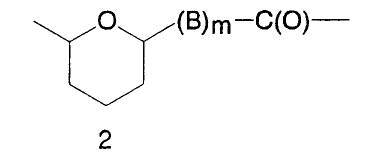 dans laquelle :
- B représente -CH₂-O-CH₂- ; et
- m vaut 0 ou 1 ;
- Z est un groupe espaceur ; et
- T est une fraction support.

2. Conjugué selon la revendication 1, dans lequel :
(i) Z représente -NH- et T-Z provient d'un support de formule T-NH₂ portant au moins un groupe amino libre, ou
(ii) Z représente NH-[D]_{d}-N=CH, où d est un entier de 0 à 2, [D] représente -NH-CO(CH₂)ₙCO-NH- (n vaut 2 ou 4) et T-Z provient d'un support de formule T-CHO portant au moins un groupe formyle ou T[COOH]ₐ où a est tel que défini ci-dessus, ou
(iii)Z est une fraction pipérazinylcarbonyle ou un groupe de formule -NH-[D]_{d}-NH-CO- où [D] et d sont tels que définis ci-dessus et T-Z provient d'un support de formule T-[COOH]ₐ tel que défini ci-dessus.

3. Conjugué selon la revendication 1, dans lequel l'anthracycline portant au moins un groupe hydroxyle primaire ou secondaire a la formule 3 : 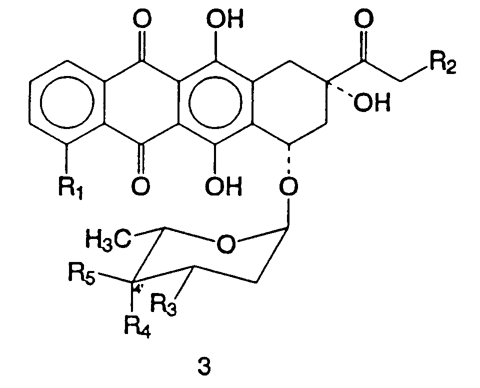 dans laquelle :
- R₁ est un atome d'hydrogène, un groupe hydroxy ou un groupe méthoxy ;
(i) R₂ est un groupe hydroxy, et R₄ ou R₅ représente un atome d'hydrogène, et l'autre parmi R₄ et R₅ est un groupe hydroxy, R₄ est un atome d'iode et R₅ est un atome d'hydrogène, ou R₄ et R₅ représentent tous deux des atomes d'hydrogène, ou
(ii) R₂ est un atome d'hydrogène et R₄ ou R₅ représente un groupe hydroxy et l'autre parmi R₄ et R₅ est un atome d'hydrogène ;
- R₃ est un groupe amino ou représente un atome d'azote enfermé dans un cycle morpholino.

4. Conjugué selon la revendication 3, dans lequel la fraction A-O- représente : 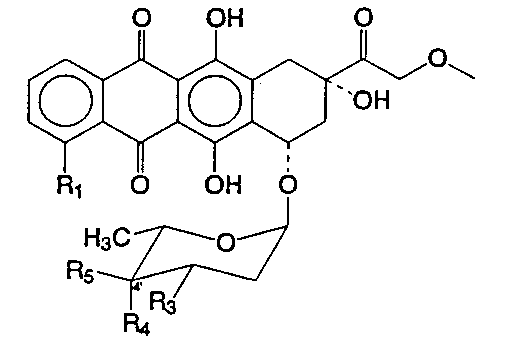 dans laquelle :
- R₁ et R₃ sont tels que définis à la revendication 3 ; et
- R₄ ou R₅ représente un atome d'hydrogène et l'autre parmi R₄ et R₅ est un groupe hydroxy, ou R₄ est un atome d'hydrogène ou d'iode et R₅ est un atome d'hydrogène.

5. Conjugué selon la revendication 3, dans lequel la fraction A-O- représente : 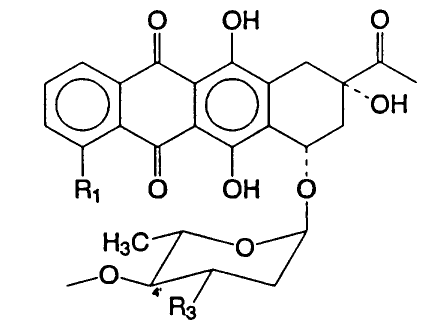 dans laquelle R₁ et R₃ sont tels que définis à la revendication 2.

6. Conjugué selon l'une quelconque des revendications précédentes, dans lequel le support est choisi parmi un anticorps polyclonal, ou un fragment de celui-ci comprenant un site de liaison aux antigènes, capable de se lier à un antigène associé à une tumeur ; un anticorps monoclonal, ou un fragment de celui-ci comprenant un site de liaison aux antigènes, capable de se lier à un antigène préférentiellement ou sélectivement exprimé sur des populations de cellules tumorales ; un peptide ou une protéine capable de se lier préférentiellement ou sélectivement à une cellule tumorale ; et un support polymère.

7. Conjugué selon la revendication 6, dans lequel le support est choisi parmi un anticorps anti-lymphocyte T, un anticorps anti-CD5, un anticorps anti-récepteur de transferrine, un anticorps anti-mélanome, un anticorps anti-marqueur de carcinome, un anticorps anti-carcinome ovarien, un anticorps anti-carcinome du sein et un anticorps anti-carcinome de la vessie.

8. Conjugué selon la revendication 6, dans lequel le support est un facteur de croissance.

9. Procédé pour la préparation d'un conjugué de formule 1 défini à la revendication 1, lequel procédé comprend :
(a) la conversion d'un dérivé de formule 4 : 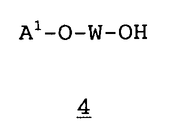 dans laquelle :
- A¹-O- est le reste d'une anthracycline portant au moins un groupe hydroxyle primaire ou secondaire et ayant le groupe amino de la fraction sucre protégé ou remplacé par un groupe morpholino ; et
- W est tel que défini à la revendication 1,
en un dérivé activé de formule 5 : 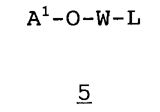 dans laquelle :
- A¹-O- et W sont tels que définis ci-dessus ; et
- L représente un groupe activateur pour former une liaison amidique ; et
(b)
(i) la condensation des composés résultants de formule 5, définis ci-dessus, avec un composé de formule T-NH₂ défini à la revendication 2, ou
(ii) la réaction du composé activé de formule 5, avec un dérivé de formule NH₂-[D]_{d}NH₂ où D et d sont tels que définis à la revendication 2, la déprotection facultative du dérivé résultant de formule 6 : 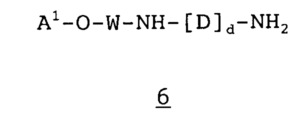 dans laquelle A¹-O-, W, d et [D] sont tels que définis à la revendication 2, et sa condensation avec un composé de formule T-CHO ou T-[COOH]ₐ défini à la revendication 2, ou
(iii)la réaction du composé de formule 5 avec une 1,4-pipérazine et la condensation du composé résultant de formule 7 : 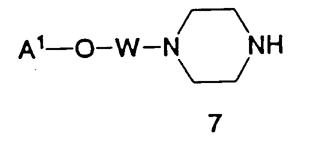 dans laquelle A¹-O- et W sont tels que définis ci-dessus,
avec un composé de formule T[COOH]ₐ défini ci-dessus, facultativement en présence d'un agent de condensation, pour donner un conjugué de formule 1, dans lequel les groupes carboxyle activés facultativement présents n'ayant pas réagi peuvent être désactivés par une amine pharmaceutiquement acceptable.

10. Composition pharmaceutique comprenant un support ou diluant pharmaceutiquement acceptable et, en tant qu'ingrédient actif, un conjugué tel que défini à l'une des revendications 1 à 8, ou qui a été préparé par un procédé tel que défini à la revendication 9.

11. Dérivé de formule 4 défini à la revendication 9.

12. Dérivé selon la revendication 11, choisi parmi :
• la 4'-épi-4'-O-(2-carboxytétrahydropyran-6-yl)-3'-désamino-3'-(4-morpholino)-daunorubicine ;
• la 14-O-(2-carboxytétrahydropyrann-6-yl)-3'-désamino-3'-(2-méthoxy-4-morpholino)-doxorubicine ; et
• la 14-O-(2-carboxyméthyloxyméthyl-tétrahydropyrann-6-yl)-3'-désamino-3'-(4-morpholino)-doxorubicine.

13. Procédé de préparation d'un dérivé de formule 4 défini à la revendication 11, lequel procédé comprend :
(a) la condensation d'une anthracycline facultativement protégée de formule A-O-H définie à la revendication 1, à condition que le groupe amino de la fraction sucre de l'anthracycline A-O-H soit sous la forme d'un groupe amino libre ou protégé, avec un dérivé dihydropyranne carboxylique de formule 8 : 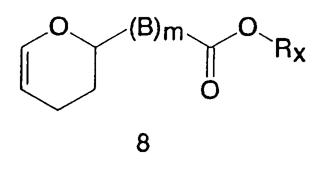 dans laquelle :
- B et m sont tels que définis à la revendication 1 ; et
- Rₓ est un groupe protecteur ;
(b) l'élimination du ou de chaque groupe protecteur à partir de l'intermédiaire résultant, pour former ainsi un dérivé de formule 4' : 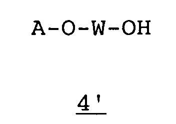 dans laquelle A-O- et W sont tels que définis à la revendication 1, à condition que le groupe amino de la fraction sucre du résidu anthracycline A-O- soit sous la forme d'un groupe amino libre ; et
(c)
(i) la conversion du groupe amino libre de la fraction sucre de l'anthracycline de formule 4' en un dérivé morpholino, ou
(ii) la protection dudit groupe amino libre de ladite anthracycline de formule 4'.

14. Dérivé de formule 4' défini à la revendication 13, à condition que, lorsque l'anthracycline a la formule 3 définie à la revendication 3, l'anthracycline n'est pas liée au groupe -O-W-OH par l'intermédiaire d'un groupe hydroxy au niveau de R₄.

15. Dérivé selon la revendication 14, choisi parmi :
• la 4'-épi-4'-O-(2-carboxytétrahydropyrann-6-yl)-daunorubicine ;
• la 14-O-(2-carboxytétrahydropyrann-6-yl)-doxorubicine ; et
• la 14-O-(2-carboxyméthyloxyméthyl-tétrahydropyrann-6-yl)-doxorubicine.

16. Procédé de préparation d'un dérivé de formule 4' défini à la revendication 14, lequel procédé comprend :
(a) la condensation d'une anthracycline facultativement protégée de formule A-O-H définie à la revendication 1, à condition que le groupe amino de la fraction sucre de l'anthracycline A-O-H soit sous la forme d'un groupe amino libre ou protégé, avec un dérivé dihydropyranne carboxylique de formule 8 défini à la revendication 13; et
(b) l'élimination du ou de chaque groupe protecteur à partir de l'intermédiaire résultant.

17. Dérivé de formule 5 défini à la revendication 9.

18. Dérivé selon la revendication 17, choisi parmi :
• la 4'-épi-4'-O-(2-succinimidocarbonyl-tétrahydropyrann-6-yl)-3'-désamino-3'-(4-morpholino)-daunorubicine ;
• la 14-O-(2-succinimidocarbonyl-tétrahydropyrann-6-yl)-3'-désamino-3'-(2-méthoxy-4-morpholino)-doxorubicine; et
• la 14-O-(2-succinimidocarbonylméthyloxyméthyl-tétrahydropyrann-6-yl)-3'-désamino-3'-(4-morpholino)-doxorubicine.

19. Procédé de préparation d'un dérivé de formule 5 défini à la revendication 17, lequel procédé comprend la conversion d'un dérivé de formule 4 telle que définie à la revendication 9 en un dérivé précité correspondant de formule 5.

20. Dérivé de formule 6 ou 7 défini à la revendication 9.

21. Procédé de préparation d'un dérivé de formule 6 ou 7 défini à la revendication 20, lequel procédé comprend :
(i) la réaction d'un dérivé de formule 5 défini à la revendication 9 avec un dérivé de formule NH₂[D]_{d}NH₂ ou la 1,4-pipérazine et, si on le désire, la déprotection du dérivé résultant de formule 6 ou 7 ainsi obtenu.

22. Composé de formule : 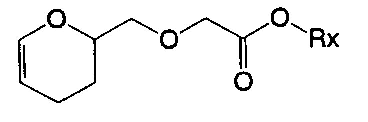 où Rₓ est un groupe protecteur.

23. Dérivé selon la revendication 22, choisi parmi :
• le 2-éthoxycarbonylméthyloxyméthyl-3,4-dihydro-2H-pyranne ; et
• le 2-méthoxycarbonylméthyloxyméthyl-3,4-dihydro-2H-pyranne.

24. Conjugué de formule 1 défini à la revendication 1, destiné à être utilisé en tant qu'agent anti-tumoral.

## Revendications (Revendications pour l'(les) Etat(s) contractant(s) suivant(s): ES)

1. Procédé pour la préparation d'un conjugué de formule 1 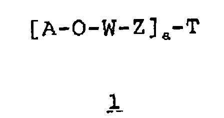 dans laquelle :
- la fraction A-O- est la reste de n'importe quelle anthracycline de formule A-O-H portant au moins un groupe hydroxyle primaire ou secondaire ;
- a est un entier de 1 à 30 ;
- W est un reste de formule 2 : 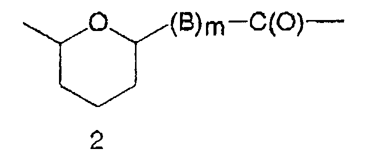 dans laquelle :
- B représente -CH₂-O-CH₂- ; et
- m vaut 0 ou 1 ;
- Z est un groupe espaceur ; et
- T est une fraction support, lequel procédé comprend :
(a) le conversion d'un dérivé de formule 4 : 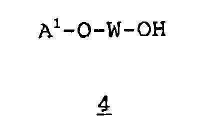 dans laquelle :
- A¹-O- est le reste d'une anthracycline portant au moins un groupe hydroxyle primaire ou secondaire et ayant le groupe amino de la fraction sucre protégé ou remplacé par un groupe morpholino ; et
- W est tel que défini ci-dessus,
en un dérivé activé de formule 5 : 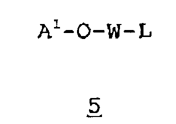 dans laquelle :
- A¹-O- et W sont tels que définis ci-dessus ; et
- L représente un groupe activateur pour former une liaison amidique ; et
(b)
(i) la condensation des composés résultants de formule 5, définis ci-dessus, avec un support de formule T-NH₂ portant au moins un groupe amino libre, ou
(ii) la réaction du composé activé de formule 5, avec un dérivé de formule NH₂-[D]_{d}NH₂ où d est un entier de 0 à 2, [D] représente -NH-CO(CH₂)ₙCO-NH- (n vaut 2 ou 4), la déprotection facultative du dérivé résultant de formule 6 : 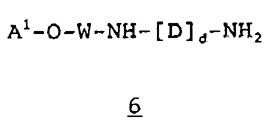 dans laquelle A¹-O, W, d et [D] sont tels que définis ci-dessus, et sa condensation avec un composé de formule T-CHO, portant au moins un groupe formyle ou T[COOH]ₐ où a est tel que défini ci-dessus, ou
(iii)la réaction du composé de formule 5 avec une 1,4-pipérazine et la condensation du composé résultant de formule 7 : 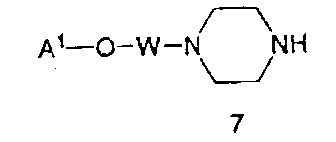 dans laquelle A¹-O- et W sont tels que définis ci-dessus,
avec un composé de formule T[COOH]ₐ défini ci-dessus, facultativement en présence d'un agent de condensation, pour donner un conjugué de formule 1, dans lequel les groupes carboxyle activés facultativement présents n'ayant pas réagi peuvent être désactivés par une amine pharmaceutiquement acceptable.

2. Procédé selon la revendication 1, dans lequel l'anthracycline portant au moins un groupe hydroxyle primaire ou secondaire a la formule 3 : 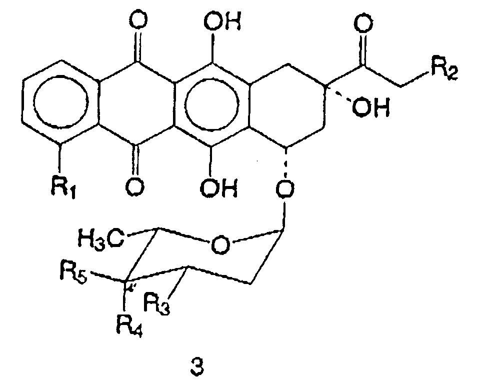 dans laquelle :
- R₁ est un atome d'hydrogène, un groupe hydroxy ou un groupe méthoxy ;
(i) R₂ est un groupe hydroxy, et R₄ ou R₅ représente un atome d'hydrogène, et l'autre parmi R₄ et R₅ est un groupe hydroxy, R₄ est un atome d'iode et R₅ est un atome d'hydrogène, ou R₄ et R₅ représentent tous deux des atomes d'hydrogène, ou
(ii) R₂ est un atome d'hydrogène et R₄ ou R₅ représente un groupe hydroxy et l'autre parmi R₄ et R₅ est un atome d'hydrogène ;
- R₃ est un groupe amino ou représente un atome d'azote enfermé dans un cycle morpholino.

3. Procédé selon la revendication 2, dans lequel la fraction A-O- représente : 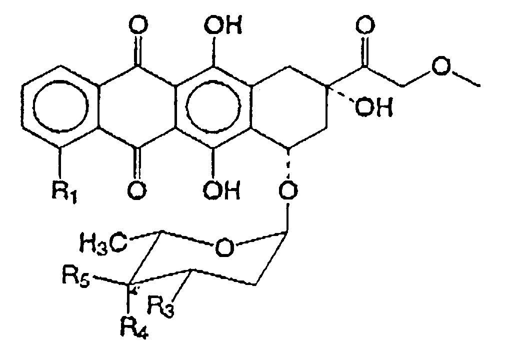 dans laquelle :
- R₁ et R₃ sont tels que définis à la revendication 2 ; et
- R₄ ou R₅ représente un atome d'hydrogène et l'autre parmi R₄ et R₅ est un groupe hydroxy, ou R₄ est un atome d'hydrogène ou d'iode et R₅ est un atome d'hydrogène.

4. Procédé selon la revendication 2, dans lequel la fraction A-O- représente : 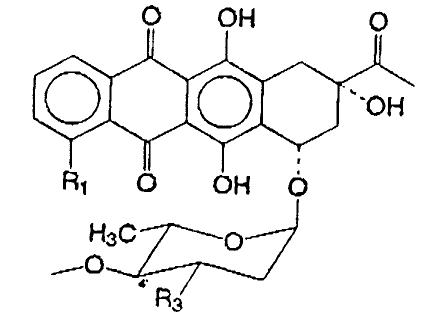 dans laquelle R₁ et R₃ sont tels que définis à la revendication 2.

5. Procédé selon l'une quelconque des revendications précédentes, dans lequel le support est choisi parmi un anticorps polyclonal, ou un fragment de celui-ci comprenant un site de liaison aux antigènes, capable de se lier à un antigène associé à une tumeur ; un anticorps monoclonal, ou un fragment de celui-ci comprenant un site de liaison aux antigènes, capable de se lier à un antigène préférentiellement ou sélectivement exprimé sur des populations de cellules tumorales ; un peptide ou une protéine capable de se lier préférentiellement ou sélectivement à une cellule tumorale ; et un support polymère.

6. Procédé salon la revendication 5, dans lequel le support est choisi parmi un anticorps anti-lymphocyte T, un anticorps anti-CD5, un anticorps anti-récepteur de transferrine, un anticorps anti-mélanome, un anticorps anti-marqueur de carcinome, un anticorps anti-carcinome ovarien, un anticorps anti-carcinome du sein et un anticorps anti-carcinome de la vessie.

7. Procédé selon la revendication 5, dans lequel le support est un facteur de croissance.

8. Procédé pour la préparation d'une composition pharmaceutique, comprenant le mélange d'un support ou diluant pharmaceutiquement acceptable et, en tant qu'ingrédient actif, d'un conjugué qui a été préparé par un procédé tel que défini dans l'une quelconque des revendications 1 à 7.

9. Procédé de préparation d'un dérivé de formule 4 défini à la revendication 1, lequel procédé comprend :
(a) la condensation d'une anthracycline facultativement protégée de formule A-O-H définie à la revendication 1, à condition que le groupe amino de la fraction sucre de l'anthracycline A-O-H soit sous la forme d'un groupe amino libre ou protégé, avec un dérivé dihydropyranne carboxylique de formule 8 : 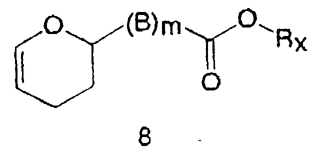 dans laquelle :
- B et m sont tels que définis à la revendication 1 ; et
- Rₓ est un groupe protecteur ;
(b) l'élimination du ou de chaque groupe protecteur à partir de l'intermédiaire résultant, pour former ainsi un dérivé de formule 4' : 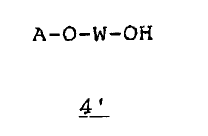 dans laquelle A-O- et W sont tels que définis à la revendication 1, à condition que le groupe amino de la fraction sucre du résidu anthracycline A-O- soit sous la forme d'un groupe amino libre ; et
(c)
(i) la conversion du groupe amino libre de la fraction sucre de l'anthracycline de formule 4' en un dérivé morpholino, ou
(ii) la protection dudit groupe amino libre de ladite anthracycline de formule 4'.

10. Procédé selon la revendication 9 dans lequel le dérivé de formule 4 est choisi parmi :
• la 4'-épi-4'-O-(2-carboxytétrahydropyran-6-yl)-3'-désamino-3'-(4-morpholino)-daunorubicine ;
• la 14-O-(2-carboxytétrahydropyrann-6-yl)-3'-désamino-3'-(2-méthoxy-4-morpholino)-doxorubicine ; et
• la 14-O-(2-carboxyméthyloxyméthyl-tétrahydropyrann-6-yl)-3'-désamino-3'-(4-morpholino)-doxorubicine.

11. Procédé de préparation d'un dérivé de formule 4' défini à la revendication 9, lequel procédé comprend :
(a) la condensation d'une anthracycline facultativement protégée de formule A-O-H définie à la revendication 1, à condition que le groupe amino de la fraction sucre de l'anthracycline A-O-H soit sous la forme d'un groupe amino libre ou protégé, avec un dérivé dihydropyranne carboxylique de formule 8 défini à la revendication 9 ; et
(b) l'élimination du ou de chaque groupe protecteur à partir de l'intermédiaire résultant.

12. Procédé selon la revendication 1, dans lequel le dérivé de formule 4' est choisi parmi :
• la 4'-épi-4'-O-(2-carboxytétrahydropyrann-6-yl)-daunorubicine ;
• la 14-O-(2-carboxytétrahydropyrann-6-yl)-doxorubicine ; et
• la 14-O-(2-carboxyméthyloxyméthyl-tétrahydropyrann-6-yl)-doxorubicine.

13. Procédé de préparation d'un dérivé de formule 5 défini à la revendication 1, lequel procédé comprend la conversion d'un dérivé de formule 4 telle que définie à la revendication 1 en un dérivé précité correspondant de formule 5.

14. Procédé selon la revendication 13, dans lequel le dérivé de formule 5 est choisi parmi :
• la 4'-épi-4'-O-(2-succinimidocarbonyl-tétrahydropyrann-6-yl)-3'-désamino-3'-(4-morpholino)-daunorubicine ;
• la 14-O-(2-succinimidocarbonyl-tétrahydropyrann-6-yl)-3'-désamino-3'-(2-méthoxy-4-morpholino)-doxorubicine; et
• la 14-O-(2-succinimidocarbonylméthyloxyméthyl-tétrahydropyrann-6-yl)-3'-désamino-3'-(4-morpholino)-doxorubicine.

15. Procédé de préparation d'un dérivé de formule 6 ou 7 défini à la revendication 1, lequel procédé comprend :
(i) la réaction d'un dérivé de formule 5 défini à la revendication 1 avec un dérivé de formule NH₂[D]_{d}NH₂ ou la 1,4-pipérazine et, si on le désire, la déprotection du dérivé résultant de formule 6 ou 7 ainsi obtenu.

## Revendications (Revendications pour l'(les) Etat(s) contractant(s) suivant(s): GR)

1. Procédé pour la préparation d'un conjugué de formule 1 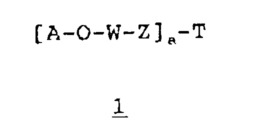 dans laquelle :
- la fraction A-O- est le reste de n'importe quelle anthracycline de formule A-O-H portant au moins un groupe hydroxyle primaire ou secondaire ;
- a est un entier de 1 à 30 ;
- W est un reste de formule 2 : 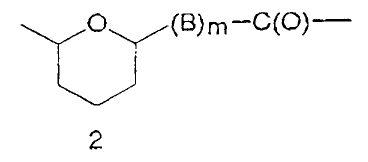 dans laquelle :
- B représente -CH₂-O-CH₂- ; et
- m vaut 0 ou 1 ;
- Z est un groupe espaceur ; et
- T est une fraction support, lequel procédé comprend :
(a) la conversion d'un dérivé de formule 4 : 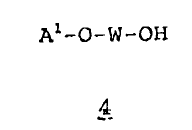 dans laquelle :
- A¹-O- est le reste d'une anthracycline portant au moins un groupe hydroxyle primaire ou secondaire et ayant le groupe amino de la fraction sucre protégé ou remplacé par un groupe morpholino ; et
- W est tel que défini ci-dessus,
en un dérivé activé de formule 5 : 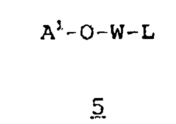 dans laquelle :
- A¹-O- et W sont tels que définis ci-dessus ; et
- L représente un groupe activateur pour former une liaison amidique ; et
(b)
(i) la condensation des composée résultants de formule 5, définis ci-dessus, avec un support de formule T-NH₂ portant au moins un groupe amino libre, ou
(ii) la réaction du composé activé de formule 5, avec un dérivé de formule NH₂-[D]_{d}NH₂ où d est un entier de 0 à 2, [D] représente -NH-CO(CH₂)ₙCO-NH- (n vaut 2 ou 4), la déprotection facultative du dérivé résultant de formule 6 : 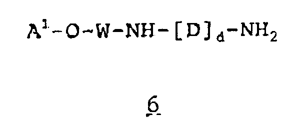 dans laquelle A¹-O-, W, d et [D] sont tels que définis ci-dessus, et sa condensation avec un composé de formule T-CHO, portant au moins un groupe formyle ou T[COOH]ₐ où a est tel que défini ci-dessus, ou
(iii)la réaction du composé de formule 5 avec une 1,4-pipérazine et la condensation du composé résultant de formule 7 : 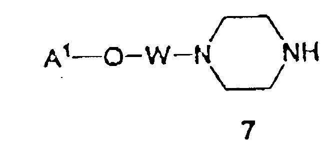 dans laquelle A¹-O- et W sont tels que définis ci-dessus,
avec un composé de formule T[COOH]ₐ défini ci-dessus, facultativement en présence d'un agent de condensation, pour donner un conjugué de formule 1, dans lequel les groupes carboxyle activés facultativement présents n'ayant pas réagi peuvent être désactivés par une amine pharmaceutiquement acceptable.

2. Procédé selon la revendication 1, dans lequel l'anthracycline portant au moins un groupe hydroxyle primaire ou secondaire a la formule 3 : 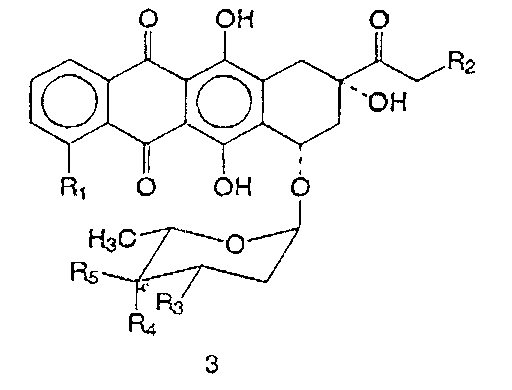 dans laquelle :
- R₁ est un atome d'hydrogène, un groupe hydroxy ou un groupe méthoxy ;
(i) R₂ est un groupe hydroxy, et R₄ ou R₅ représente un atome d'hydrogène, et l'autre parmi R₄ et R₅ est un groupe hydroxy, R₄ est un atome d'iode et R₅ est un atome d'hydrogène, ou R₄ et R₅ représentent tous deux des atomes d'hydrogène, ou
(ii) R₂ est un atome d'hydrogène et R₄ ou R₅ représente un groupe hydroxy et l'autre parmi R₄ et R₅ est un atome d'hydrogène ;
- R₃ est un groupe amino ou représente un atome d'azote enfermé dans un cycle morpholino.

3. Procédé selon la revendication 2, dans lequel la fraction A-O- représente : 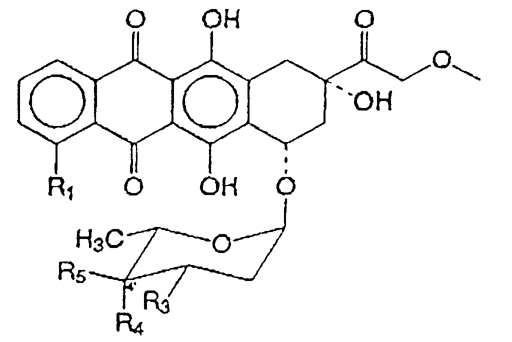 dans laquelle :
- R₁ et R₃ sont tels que définis à la revendication 2 ; et
- R₄ ou R₅ représente un atome d'hydrogène et l'autre parmi R₄ et R₅ est un groupe hydroxy, ou R₄ est un atome d'hydrogène ou d'iode et R₅ est un atome d'hydrogène.

4. Procédé selon la revendication 2, dans lequel la fraction A-O- représente : 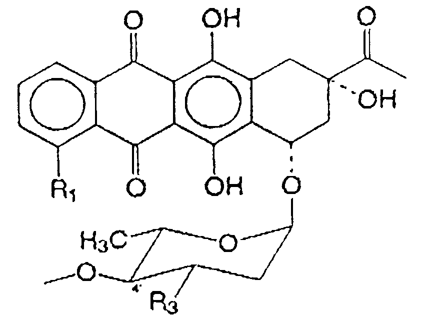 dans laquelle R₁ et R₃ sont tels que définis à la revendication 2.

5. Procédé selon l'une quelconque des revendications précédentes, dans lequel le support est choisi parmi un anticorps polyclonal, ou un fragment de celui-ci comprenant un site de liaison aux antigènes, capable de se lier à un antigène associé à une tumeur ; un anticorps monoclonal, ou un fragment de celui-ci comprenant un site de liaison aux antigènes, capable de se lier à un antigène préférentiellement ou sélectivement exprimé sur des populations de cellules tumorales ; un peptide ou une protéine capable de se lier préférentiellement ou sélectivement à une cellule tumorale ; et un support polymère.

6. Procédé selon la revendication 5, dans lequel le support est choisi parmi un anticorps anti-lymphocyte T, un anticorps anti-CD5, un anticorps anti-récepteur de transferrine, un anticorps anti-mélanome, un anticorps anti-marqueur de carcinome, un anticorps anti-carcinome ovarien, un anticorps anti-carcinome du sein et un anticorps anti-carcinome de la vessie.

7. Procédé selon la revendication 5, dans lequel le support est un facteur de croissance.

8. Procédé pour la préparation d'une composition pharmaceutique, comprenant le mélange d'un support ou diluant pharmaceutiquement acceptable et, en tant qu'ingrédient actif, d'un conjugué qui a été préparé par un procédé tel que défini dans l'une quelconque des revendications 1 à 7.

9. Dérivé de formule 4 défini à la revendication 1.

10. Dérivé selon la revendication 9, choisi parmi :
• la 4'-épi-4'-O-(2-carboxytétrahydropyran-6-yl)-3'-désamino-3'-(4-morpholino)-daunorubicine ;
• la 14-O-(2-carboxytétrahydropyrann-6-yl)-3'-désamino-3'-(2-méthoxy-4-morpholino)-doxorubicine ; et
• la 14-O-(2-carboxyméthyloxyméthyl-tétrahydropyrann-6-yl)-3'-désamino-3'-(4-morpholino)-doxorubicine.

11. Procédé de préparation d'un dérivé de formule 4 défini à la revendication 9, lequel procédé comprend :
(a) la condensation d'une anthracycline facultativement protégée de formule A-O-H définie à la revendication 1 à condition que le groupe amino de la fraction sucre de l'anthracycline A-O-H soit sous la forme d'un groupe amino libre ou protégé, avec un dérivé dihydropyranne carboxylique de formule 8 : 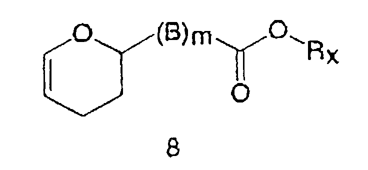 dans laquelle :
- B et m sont tels que définis à la revendication 1 ; et
- Rₓ est un groupe protecteur ;
(b) l'élimination du ou de chaque groupe protecteur à partir de l'intermédiaire résultant, pour former ainsi un dérivé de formule 4' : 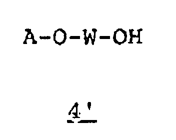 dans laquelle A-O- et W sont tels que définis à la revendication 1, à condition que le groupe amino de la fraction sucre du résidu anthracycline A-O- soit sous la forme d'un groupe amino libre ; et
(c)
(i) la conversion du groupe amino libre de la fraction sucre de l'anthracycline de formule 4' en un dérivé morpholino, ou
(ii) la protection dudit groupe amino libre de ladite anthracycline de formule 4'.

12. Dérivé de formule 4' défini à la revendication 11, à condition que, lorsque l'anthracycline a la formule 3 définie à la revendication 2, l'anthracycline n'est pas liée au groupe -O-W-OH par l'intermédiaire d'un groupe hydroxy au niveau de R₄.

13. Dérivé selon la revendication 12, choisi parmi :
• la 4'-épi-4'-O-(2-carboxytétrahydropyrann-6-yl)-daunorubicine ;
• la 14-O-(2-carboxytétrahydropyrann-6-yl)-doxorubicine ; et
• la 14-O-(2-carboxyméthyloxyméthyl-tétrahydropyrann-6-yl)-doxorubicine.

14. Procédé de préparation d'un dérivé de formule 4' défini à la revendication 12, lequel procédé comprend :
(a) la condensation d'une anthracycline facultativement protégée de formule A-O-H définie à la revendication 1, à condition que le groupe amino de la fraction sucre de l'anthracycline A-O-H soit sous la forme d'un groupe amino libre ou protégé, avec un dérivé dihydropyranne carboxylique de formule 8 défini à la revendication 11 ; et
(b) l'élimination du ou de chaque groupe protecteur à partir de l'intermédiaire résultant.

15. Dérivé de formule 5 défini à la revendication 1.

16. Dérivé selon la revendication 15, choisi parmi :
• la 4'-épi-4'-O-(2-succinimidocarbonyl-tétrahydropyrann-6-yl)-3'-désamino-3'-(4-morpholino)-daunorubicine ;
• la 14-O-(2-succinimidocarbonyl-tétrahydropyrann-6-yl)-3'-désamino-3'-(2-methoxy-4-morpholino)-doxorubicine; et
• la 14-O-(2-succinimidocarbonylméthyloxyméthyl-tétrahydropyrann-6-yl)-3'-désamino-3'-(4-morpholino)-doxorubicine.

17. Procédé de préparation d'un dérivé de formule 5 défini à la revendication 15, lequel procédé comprend la conversion d'un dérivé de formule 4 telle que définie à la revendication 1 en un, dérivé précité correspondant de formule 5.

18. Dérivé de formule 6 ou 7 défini a la revendication 1.

19. Procédé de préparation d'un dérivé de formule 6 ou 7 défini à la revendication 18, lequel procédé comprend :
(i) la réaction d'un dérivé de formule 5 défini à la revendication 1 avec un dérivé de formule NH₂[D]_{d}NH₂ ou la 1,4-pipérazine et, si on le désire, la déprotection du dérivé résultant de formule 6 ou 7 ainsi obtenu.

20. Composé de formule : 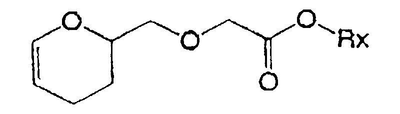 où Rₓ est un groupe protecteur.

21. Dérivé selon la revendication 20, choisi parmi :
• le 2-éthoxycarbonylméthyloxyméthyl-3,4-dihydro-2H-pyranne ; et
• le 2-méthoxycarbonylméthyloxyméthyl-3,4-dihydro-2H-pyranne.
